# EUROPÄISCHE PATENTSCHRIFT

(11) **EP 0 818 460 B1**
(45) Veröffentlichungstag und Bekanntmachung des Hinweises auf die Patenterteilung: **15.01.2003**
(21) Anmeldenummer: 97111426.9
(22) Anmeldetag: 07.07.1997
(51) Int. Cl.: C07H 21/00, C07H 19/20, C07H 19/10

(54) **Festphasensynthese von Oligonucleotiden**
Synthesis of Oligonucleotides on a solid support.
Synthèse d'oligonucléotides sur support solide.

(30) Priorität: 11.07.1996 DE 19627898
(43) Veröffentlichungstag der Anmeldung: 14.01.1998
(73) Patentinhaber: Aventis Pharma Deutschland GmbH, 65929 Frankfurt am Main (DE)
(72) Erfinder: Pfleiderer, Wolfgang, Prof. Dr., 78464 Konstanz (DE); Beier, Markus, 75417 Mühlacker (DE)

(56) Entgegenhaltungen:
- HATA, T. ET AL.: "Cyclic diacyl groups for protection of the N6-amino group of deoxyadenosine in oligodeoxynucleotide synthesis" NUCLEIC ACIDS RES., Bd. 12, Nr. 22, 1984, Seiten 8525-38, XP002088352
- MISRA, K. ET AL.: "Naphthaloyl group: a new selective amino protecting group for deoxynucleosides in oligonucleotide synthesis" CAN. J. CHEM., Bd. 66, Nr. 12, 1988, Seiten 2989-94, XP002088353
- ISHIDO, Y. ET AL.: "An efficient method for the synthesis of (4-15N) cytidine and (6-N15) adenosine derivatives" TETRAHEDR .LETT., Bd. 36, Nr. 1, 1995, Seiten 91-4, XP002088354
- BEIER, M. UND PFLEIDERER, W.: "Phthaloyl strategy. A new approach towards oligodeoxyribonucleotide synthesis" NUCLEOSIDES, NUCLEOTIDES, Bd. 16, Nr. 7-9, 1997, Seiten 1621-4, XP002088355

## Beschreibung

Die chemische Polykondensation von Mononucleotiden ist eine wichtige Methode zur Herstellung von Desoxyribonucleinsäure (DNA) oder Ribonucleinsäure (RNA).

Oligonucleotide finden in wachsendem Maße Anwendung als Inhibitoren der Genexpression (J. F. Milligan, M. D. Matteucci und J. C. Martin, J. Med. Chem. 36 (1993) 1923; E. Uhlmann and A. Peyman, Chemical Reviews 90 (1990) 543) oder als Ribozyme (z.B. D. Castanotto, J. J. Rossi, J. O. Deshler, Critical Rev. Eukar. Gene Expr. 2 (1992) 331), oder in der Diagnostik als DNA-Sonden (z.B. Beck und Köster, Anal. Chem. 62 (1990) 2258). Es besteht daher ein großer Bedarf nach geeigneten Verfahren zur Synthese solcher Verbindungen.

Eine Übersicht über den Stand der Technik der Oligonucleotidsynthese findet sich bei E. Sonveaux, Bioorg. Chem. 14 (1986) 274; E. Uhlmann and A. Peyman, Chemical Reviews 90 (1990) 543, Beaucage und lyer Tetrahedron 49 (1993) 10441-10488.

Ein grundlegendes Problem bei der chemischen Synthese von DNA oder RNA ist das Auffinden geeigneter Schutzgruppen der Amino- und Hydroxygruppen der Nucleosidbasen und der Zuckerreste. Diese Schutzgruppen müssen einerseits unter den Bedingungen der Polykondensationsreaktion, d.h. während der Reaktion, stabil sein und andererseits müssen sie genügend labil sein, um am Ende der Reaktion wieder entfernt werden zu können, ohne die Phosphodiester-Bindung wieder zu spalten (H. G. Khorana; Pure Appl. Chem. 17 (1968) 349].

Die gegenwärtige Praxis bei der DNA Synthese sieht im wesentlichen drei Schritte vor: (a) sequentieller Aufbau der in verschiedener Weise geschützten Nucleotide auf einem festen Träger; (b) Spaltung der synthetisierten Oligonucleotide vom Träger; (c) Entschützung der Oligonucleotide. Während der Aufbau des Oligonucleotids am festen Träger sehr schnell erfolgt -für ein 20 mer wird ungefähr eine Stunde benötigt- und auch die Abspaltung vom Träger innerhalb einer Stunde vollständig ist, bleibt die endgültige Entschützung des Oligonucleotids problematisch. Die Standard Oligonucleotid-Synthese (z.B. M. Reddy, N. B. Hanna, F. Farooqui, WO 95/24413) sieht eine Behandlung von ca. 6h bei 55 °C mit konz. NH₃ vor, wenn die etablierten Schutzgruppen Benzoyl für dA und dC, sowie Butyroyl für dG eingesetzt werden. Zur Beschleunigung dieses letzten Schritts wurden in jüngerer Zeit eine ganze Reihe von Schutzgruppen vorgeschlagen, die gegenüber der ammoniakalischen Aminolyse empfindlicher sind, als die konventionell geschützten Nucleotid-Derivate (M. Reddy et al., vide supra; Beaucage und lyer, vide supra). Diese beinhalten beispielsweise die Phenoxyacetyl-Gruppe (Schulhof et al., Nucl. Acids Res. 15 (1987) 397; die Dimethylformamidin-Gruppe (Vu et al., Tetrahedron Lett. 31 (1990) 7269, die tert.-Butylphenoxyacetyl-Gruppe (Sinha et al., Biochimie 75 (1993) 13, oder Phenylacetyl-Schutzgruppen wie in Reddy et al. -vide suprabeschrieben. Mit diesen ammoniaklabilen Schutzgruppe wird zwar die Entschützungszeit bei 55 °C auf 15-60 Minuten reduziert, doch ist deren Gebrauch auch mit Nachteilen behaftet: Die Labilität dieser Gruppen führt zunächst auch zu einer Instabilität gegenüber den DNA Synthesebedingungen, z. B. während des capping Schrittes (Beaucage und Iyer, vide supra). Phenoxyacetyl Schutzgruppen beispielsweise reduzieren die Löslichkeit der Nucleotid-Derivate, so daß Lösungsmittelgemische eingesetzt werden müssen.

Ein weiteres Kriterium für die Anwendung von Schutzgruppen für die exocyclischen Aminofunktionen der Nucleobasen ist die Reinheit der entstehenden Produkte. Bei Entschützungsprozeduren mit Ammoniak, die nach bzw. während der Abspaltung vom Träger erfolgen, wird stets ein Gemisch aus Oligonucleotid und abgespaltenen Schutzgruppen erhalten. Das Oligonucleotid muß dann in weiteren Reinigungsschritten gesäubert werden. Vorteilhafter sind Schutzgruppen, die sich noch am festen Träger abspalten lassen, ohne daß das Oligonucleotid von letzterem abgespalten wird. Eine solche Schutzgruppe ist beispielsweise die para-Nitrophenylethyloxycarbonyl-Schutzgruppe, die sich noch am Träger mit DBU entfernen läßt. Die anschließende Spaltung des Oligonucleotids vom Träger mit Ammoniak liefert schon reines Oligonucleotid (F. Himmelsbach et al., Tetrahedron 40 (1984) 59).

Ein weiteres Kriterium für die Brauchbarkeit von Schutzgruppen für die exocyclischen Aminofunktionen der Nucleobasen ist die Stabilität gegenüber sauren Bedingungen, wie sie in der Regel zur Abspaltung der 5'-Hydroxyschutzgruppe in jedem Reaktionscyclus angewandt werden, z.B. 2% Dichloressigsäure in Dichlormethan. Diese Bedingungen führen , insbesondere bei Deoxyadenosin zu einem nicht unerheblichen Anteil von Depurinierung. Cyclische Diacylgruppen wie beispielsweise Phthaloyl- oder Succinoyl-Gruppen als Schutz der exocyclischen Aminofunktion erwiesen sich als besonders stabil gegenüber den Depurinierungsbedingungen (Kume et al., Tetrahedron Lett. 23 (1982) 4365; Nucleic Acids Res. 12 (1984) 8525; Nucleic Acids Res. Symp. Ser. 11 (1982) 26; Chemistry Letters 1983, 1597). Die Entschützung dieser Gruppen erfolgte jedoch ebenfalls mit Ammoniak (Kume et al., vide supra). Weitere cyclische Diacylgruppen sind beispielsweise die Naphthaloyl-Gruppe (Dikshit et al., Can. J. Chem. 66 (1988) 2989, die ebenfalls stabil gegen Depurinierung ist und gleichfalls mit Ammoniak wieder entfernt wurde.

Überraschend wurde nun gefunden, daß diese cyclischen Diacylgruppen nicht nur besonders stabil sind gegenüber Depurinierungsbedingungen, sondern sich bequem mit einer starken, nicht nucleophilen Base wie beispielsweise DBU abspalten lassen. Dies ist um so erstaunlicher, da Phthaloyl-Gruppen teilweise unter Zuhilfenahme von DBU - allerdings bei niedrigeren DBU-Konzentrationeneingeführt wurden (Kamaike et al., Tetrahedron Lett. 36 (1995) 91). Wegen der hohen Stabilität der mit cyclischen Diacylgruppen geschützter Nucleobasen gegen Depurinierung und der Möglichkeit, diese vor der Abspaltung des Oligonucleotids vom Träger leicht zu entfernen, bieten diese sich als ideale Schutzgruppen zur Herstellung von Oligonucleotiden an.

Gegenstand der Erfindung ist daher ein Verfahren zur Herstellung von Oligonukleotiden mittels Festphasensynthese durch
a) sequentiellen Aufbau der Nucleotide an einem festen Träger nach bekannten Methoden, wobei vorhandene exocyclische Aminogruppen an den Nucleobasen durch eine cyclische Diacylgruppe geschützt sind und gegebenenfalls vorhandene Phosphatschutzgruppen durch starke, nicht nukleophile Basen abspaltbar sind,
b) Entschützung der am festen Träger gebundenen Oligonukleotide, und
c) Abspaltung der entschützten Oligonukleotide vom festen Träger nach bekannten Methoden,
dadurch gekennzeichnet, daß die am festen Träger gebundenen Oligonukleotide in Gegenwart einer starken, nicht nukleophilen Base in einem geeigneten organischen Lösungsmittel wie beispielsweise Acetonitril, Pyridin oder N-Methylimidazol entschützt werden.

Starke, nicht nukleophile Basen sind dem Fachmann bekannt, wie beispielsweise DBU (Diazabicyclo[5.4.0]undec-7-en, DABCO (Diazabicyclo[2.2.2]octan), DBN (Diazabicyclo-[4.3.0]non-5-en), Ethyldiisopropylamin, Triethylamin, N-Ethylmorpholin, DMAP (Dimethylaminopyridin), Lutidin oder ungeladene, peralkylierte Polyamino-Phosphazen-Basen (R. Schwesinger, Angew. Chem. 99 (1987) 1212). Bevorzugt ist die starke, nicht nukleophile Base DBU.

Bevorzugt erfolgt die Entschützung in Gegenwart von 0,1 bis 5 M Lösung von DBU bei 0 bis 70°C für 0,1 bis 16 h, besonders bevorzugt in Gegenwart einer 0,3 bis 3 M Lösung von DBU bei 10 bis 40°C für 0,1 bis 2h, ganz besonders bevorzugt in Gegenwart einer 0,5 bis 2,5 M Lösung von DBU bei 20 bis 30°C für 0,2 bis 1,5h.

Der Begriff "Oligonukleotide" umfaßt ganz allgemein Polydeoxyribonukleotide, die modifizierte und/oder nichtmodifizierte 2'-Deoxy-Ribose-Bausteine enthalten (DNA); Polyribonukleotide, die modifizierte und/oder nichtmodifizierte Ribose-Bausteine enthalten (RNA); aber auch andere Polynukleotide, die aus N-Glycosiden oder C-Glycosiden von modifizierten und/oder nichtmodifizierten Purin- oder Pyrimidinbasen aufgebaut sind, wobei die Phosphatbrücken der Polydeoxyribonukleotide, Polyribonukleotide oder Polynukleotide ebenfalls Modifikationen aufweisen können oder durch andere Strukturen ersetzt sind, wobei die Oligonukleotide mindestens eine Base mit einer exocyclischen Aminogruppe aufweisen.

Solche Modifikationen, die nach an sich bekannten Methoden eingeführt werden, sind beispielsweise:
a) Modifikationen der Phosphatbrücke
   Beispielhaft seien genannt: Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate, Phosphorodithioate und Methylphosphonate.
b) Ersatz der Phosphatbrücke
   Beispielhaft seien genannt: Ersatz durch Acetamid, Formacetal, 3'-Thioformacetal, Methylhydroxylamin, Oxim, Methylendimethylhydrazo, Dimethylsulfon, Silylgruppen. Bevorzugt ist der Ersatz durch Acetamid, Formacetale und 3'-Thioformacetale.
c) Modifikationen des Zuckers
   Beispielhaft seien genannt: α-anomere Zucker, 2'-O-Methylribose, O-Butylribose, 2'-O-Allylribose, 2'-Fluoro-2'-desoxyribose, 2'-Amino-2'desoxyribose, α-Arabinofuranose, Carbocyclische Zuckeranaloge. Bevorzugte Modifikation ist die durch 2'-O-Methylribose und 2'-O-n-Butylribose. Ganz besonders bevorzugt sind solche Modifikationen, bei denen die 2'- und 3'-Kohlenstoffatome der O-Ribose über eine Doppelbindung verknüpft sind und jeweils ein Wasserstoffatom als Substituenten tragen.

Das erfindungsgemäße Verfahren eignet sich somit beispielsweise zur
Herstellung von Verbindungen der Formel I 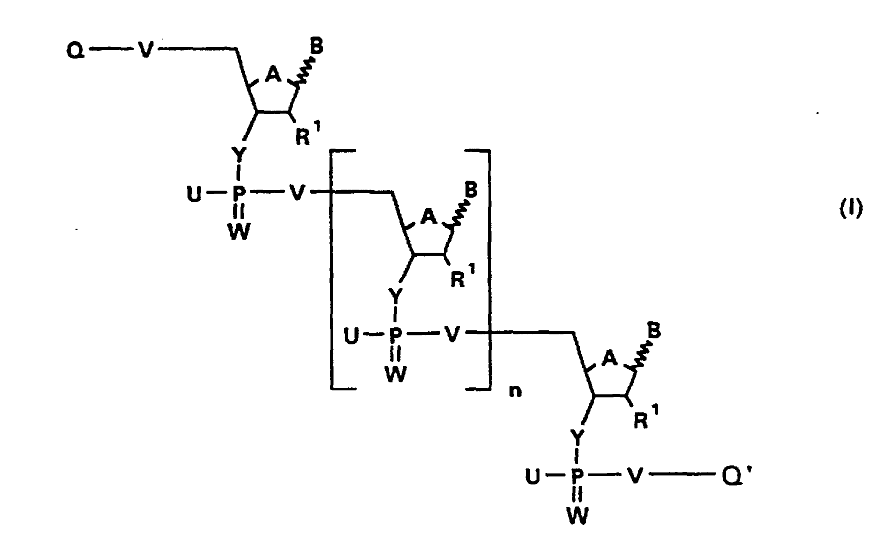 worin
- R₁: Wasserstoff, Hydroxy, C₁-C₁₈-Alkoxy, gegebenenfalls ein bis dreifach substituiert mit Hydroxy oder C₁-C₄-Alkoxy, C₁-C₄-Alkyl-O-(CH₂CH₂O)ₛ, worin s eine Zahl von 1-3 bedeutet; O-Allyl, Halogen, Azido oder Amino bedeutet;
- A: unabhängig voneinander für Oxy, Thioxy oder Methylen steht;
- W: unabhängig voneinander Oxo, Thioxo oder Selenoxo bedeutet;
- V: unabhängig voneinander Oxy, Sulfandiyl oder Imino bedeutet;
- Y: unabhängig voneinander Oxy, Sulfandiyl, Imino oder Methylen bedeutet;
- B: für eine in der Nucleotidchemie übliche Base, beispielsweise für natürliche Basen wie Adenin, Cytosin, Guanin, Uracil und Thymin oder unnatürliche Basen wie beispielsweise Purin, 2.6-Diaminopurin, 7-Deazaadenin, 7-Deazaguanin, 7-Deaza-7-C₁-C₃-alkinyl-adenin, 7-Deaza-7-C₁-C₃-alkinylguanin, N₄N₄-Ethanocytosin, N₆N₆-Ethano-2.6-diaminopurin, Pseudoisocytosin, 5-C₂-C₆-Alkinuracil, 5-C₂-C₆-Alkincytosin, bevorzugt 5-Propinuracil, 5-Propincytosin, 5-Hexinuracil, 5-Hexincytosin, oder 5-Fluorocytosin, 5-Fluoruracil, 5-Hydroxymethyluracil, 5-Bromcytosin, steht; wobei mindestens ein B für eine Base steht, die eine exocyclische Aminogruppe aufweist;
- n: eine ganze Zahl von 1 bis 100 bedeutet;
- U: Hydroxy, Mercapto, BH₃, SeH, C₁-C₁₈-Alkoxy, vorzugsweise C₁-C₆₋ Alkoxy, C₁-C₁₈-Alkyl,vorz_{ugsweise} C₁-C₆-Alkyl, C₆-C₂₀-Aryl, (C₆-C₁₄)-Aryl-(C₁-C₈)-alkyl, NHR³, NR³R⁴ oder einen Rest der Formel II

(OCH₂CH₂)ₚO(CH₂)_{q}CH₂R² (II)
bedeutet, worin
- R³: C₁-C₁₈-Alkyl, vorzugsweise C₁-C₈-Alkyl, C₆-C₂₀-Aryl, (C₆-C₁₄)-Aryl-(C₁-C₈)-alkyl, -(CH₂)_{c}-[NH(CH₂)_{c}]_{d}-NR⁵R⁵, worin c eine ganze Zahl von 2 bis 6 und d eine ganze Zahl von 0 bis 6 ist;
- R⁵: unabhängig voneinander Wasserstoff, C₁-C₆-Alkyl oder C₁-C₄-Alkoxy-C₁-C₆-alkyl, bevorzugt Methoxyethyl, ist;
- R⁴: C₁-C₁₈-Alkyl, vorzugsweise C₁-C₈-Alkyl und besonders bevorzugt C₁-C₄-Alkyl, C₆-C₂₀-Aryl oder (C₆-C₁₀)-Aryl-(C₁-C₈)-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann;
- p: eine ganze Zahl von 1 bis 100, bevorzugt 3 bis 10 ist;
- q: eine ganze Zahl von 0 bis 22, bevorzugt 0 bis 15 ist;
- R²: Wasserstoff oder eine funktionelle Gruppe wie Hydroxy, Amino, NHR₆, COOH, CONH₂, COOR₇ oder Halogen bedeutet, worin
R⁶ C₁-C₄-Alkyl und
R⁷ C₁-C₄-Alkyl, vorzugsweise Methyl bedeutet;

Q und Q' unabhängig voneinander Wasserstoff bedeuten oder für Konjugate stehen, welche die Eigenschaften von Antisense-Oligonucleotiden oder von Tripelhelix bildenden Oligonucleotiden (wie beispielsweise Zellpenetration, Nucleaseabbau, Affinität zur Target-RNA/DNA, Pharmakokinetik) günstig beeinflußen oder als Markierung einer DNA Sonde dienen oder bei der Hybridisierung des Oligonucleotidanalogons an die Target-Nucleinsäure diese unter Bindung oder Quervernetzung angreifen wie beispeisweise Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie (C₁₂-C₂₀)-Alkyl, mit Lipiden wie 1,2-Di-hexadecyl-rac-glycerin, mit Steroiden wie Cholesterin oder Testosteron, mit Vitaminen wie Vitamin E, mit Poly- bzw. Oligo-ethylengylcol, mit (C₁₂-C₁₈)-Alkyl-Phosphatdiestern, mit -O-CH₂-CH(OH)-O-(C₁₂-C₁₈)-Alkyl; oder besonders bevorzugt für Konjugate mit lipophilen Molekülen wie (C₁₂-C₂₀)-Alkyl, mit Steroiden wie Cholesterin oder Testosteron, mit Poly- oder Oligoethylenglykol, mit Vitamin E, mit Interkalatoren wie Pyren, mit (C₁₄-C₁₈)-Alkyl-Phosphatdiestern, oder mit -O-CH₂-CH(OH)-O-(C₁₂-C₁₆)-Alkyl stehen;

R¹ und der benachbarte Phosphorylrest sowohl in 2'- und 3'-Stellung oder auch umgekehrt in 3'- und 2'-Stellung befinden können, wobei jedes Nucleotid in seiner D- bzw. L-Konfiguration vorliegen kann und sich die Base B in alpha- bzw. beta-Stellung befinden kann, wobei das Oligonukleotide auch 3'-3' bzw. 5'-5'-Inversionen beinhalten kann (Ch. Chaix et al., Bioorg. & Med. Letters 6 (1996) 827); dadurch gekennzeichnet, daß man
(a) eine Verbindung der Formel III 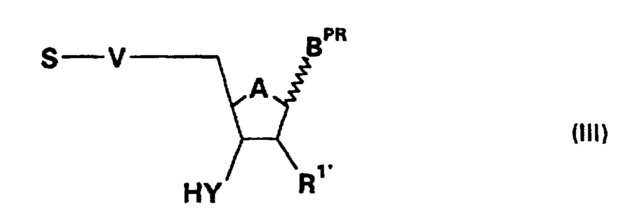 in der A, Y, V, wie oben definiert sind und
   R^{1'} wie R¹ definiert ist wobei, wenn R¹ für Hydroxy oder Amino steht R^{1'} eine entsprechend geschützte Gruppe bedeutet;
   - S: für eine im sauren abspaltbare 5'- Schutzgruppe, wie beispielsweise Dimethoxytrityl, Monomethoxytrityl, Trityl, Pixyl, bevorzugt Dimethoxytrityl und Monomethoxytrityl steht;
   - B^{PR}: für eine natürliche oder unnatürliche Nucleobase steht, wobei gegebenenfalls vorhandenen exocyclische Aminogruppen durch eine cyclische Diacylgruppe geschützt sind,
   nach bekannten Verfahren mit einer Verbindung der Formel IV umsetzt 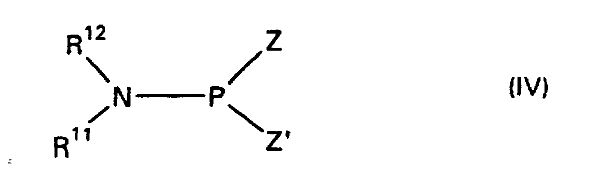 worin
   - Z': für OR¹³ oder C₁-C₁₈-Alkyl, C₁-C₁₈-Alkoxy, C₆-C₂₀-Aryl, C₆-C₁₄-Aryl-C₁-C₈-Alkyl, bevorzugt für OR¹³, C₁-C₆-Alkyl, C₁-C₆-Alkoxy, C₆-C₂₀-Aryl, C₆-C₁₄-Aryl-C₁-C₈-Alkyl, besonders bevorzugt für OR¹³ steht;
   - R¹¹ und R¹²: gleich oder verschieden sind und C₁-C₈-Alkyl, vorzugsweise Isopropyl, oder C₅-C₁₂-Cycloalkyl, vorzugsweise bis C₈, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-Alkyl-Ester, bedeuten; und
   - R¹³: für eine durch starke, nicht nukleophile Basen abspaltbare Schutzgruppe steht,
   - Z: für Chlor oder Brom oder einen Rest der Formel NR¹¹R¹², wobei R¹¹ und R¹² wie oben definiert sind, steht;
   in Gegenwart einer Base, vorzugsweise Pyridin oder einer Mischung von Tetrahydrofuran (THF), Dioxan, Dichlormethan (DCM), Chloroform, und/oder Acetonitril mit einem C₁-C₄-Trialkylamin, vorzugsweise Trimethyl-, Triethyloder Diisopropylethyl-Amin oder, wenn Z ein Rest der Formel NR¹¹R¹² ist, dann in Gegenwart einer Verbindung der Formel [HNR¹⁴R¹⁵R¹⁶]⁽⁺⁾ A⁽⁻), wobei R¹⁴, R¹⁵, R¹⁶ gleich oder verschieden voneinander sind und eine C₁-C₄-Alkylgruppe und A Fluor, Chlor, Brom, insbesondere Chlor, bedeuten, oder Tetrazol oder 5-(C₁-C₄-Alkylthio)-1H-tetrazol, 5-(C₆-C₁₂)-Aryl-1H-tetrazol, oder anderen Aktivatoren wie beispielsweise Pyridin-Hydrochlorid, vorzugsweise in Gegenwart von Tetrazol oder Pyridin-Hydrochlorid zu einer Verbindung der Formel V umsetzt 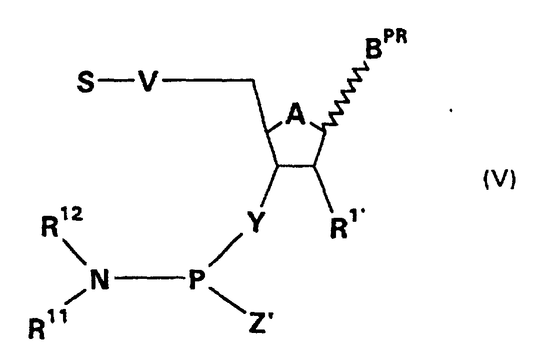 worin S, V, Y,A, B^{PR}, Z', R^{1'}, R¹¹, R¹² wie oben definiert sind;
(b) Verbindungen der Formel III nach bekannten Verfahren mit 1 bis 10 Equivalenten, bevorzugt mit 1 bis 2 Equivalenten eines Linkers, wie Bernsteinsäureanhydrid, in einem geeigneten organischen Lösungsmittel, beispielsweise Methylenchlorid, gegebenenfalls nach Zugabe eines Katalysators, beispielsweise 4-Dimethylaminopyridin, zu einer Verbindung der Formel VI 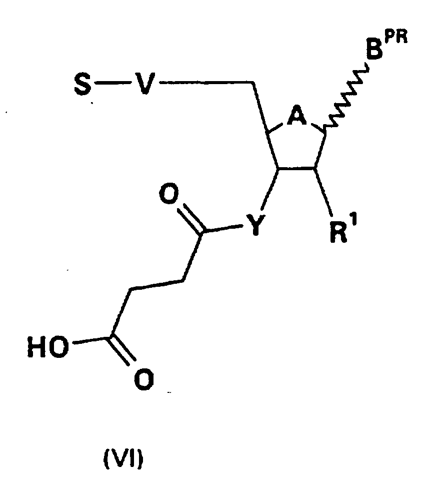 wobei S, V, Y,A, B^{PR} und R¹ wie oben definiert sind, umsetzt und anschließend nach bekannten Verfahren, wie beispielsweise Extraktion, Kristallisation, Chromatographie aufarbeitet, wobei der Bernsteinsäure-Rest in 3'-Position als Linker zu dem in der Synthese eingesetzten Polymerträger dient und alternativ zum Bernsteinsäurelinker auch andere Linker, wie z.B. in Sonveaux [Bioorg. Chem. 14 (1986) 274] beschrieben, verwendet werden können;
(c) die Verbindung der Formel VI nach bekannten Verfahren auf einen festen Träger SS, wie beispielsweise CPG® (CPG = Controlled Pore Glass) oder Tentagel®, bevorzugt DBU stabile Träger wie das "long chain methylaminoalkyl-CPG" [Stengele, Tetrahedron Lett. 1990, 31, 2549], beispielsweise durch Umsetzung mit DCC und p-Nitrophenol in einem geeigneten Lösungsmittel, oder durch Umsetzung mit TOTU (O-[(ethoxycarbonyl)cyanomethylenamino]-N,N,N',N'-tetramethyluronium tetrafluor-borat: W. König, G. Breipohl, P. Pokorny, M. Birkner, Proceedings of the 21st European Peptide Symposium 1990, E. Giralt, D. Andreu, Eds., ESCOM, Leiden, S. 143.), gegebenenfalls unter Zusatz einer geeigneten Base wie beispielsweise N-Methylmorpholin, N-Ethylmorpholin oder Ethyldiisopropylamin oder Triethylamin, in einem geeigneten Lösungsmittel koppelt [wie z.B.in M.J. Gait, Oligonucleotide Synthesis - a practical approach, IRL Press, 1984 beschrieben], wobei Verbindungen der Formel VII erhalten werden; 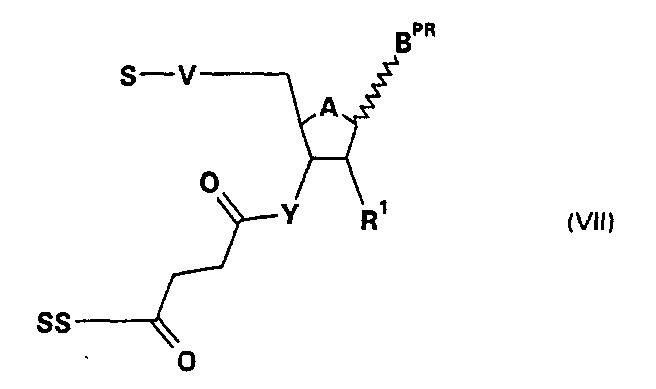
(d) die 5'-Schutzgruppe von VII nach bekannten Verfahren, beispielsweise durch Behandlung mit 1 - 4%iger Di- oder Trichloressigsäure in Methylenchlorid oder Chloroform, abspaltet;
(e) die erhaltene Verbindung mit einer Verbindung der Formel V in einem geeigneten organischen Lösungsmittel, bevorzugt Acetonitril, in Gegenwart einer Verbindung der Formel [HNR¹⁴R¹⁵R¹⁶]⁽⁺⁾ A⁽⁻⁾ ,die wie oben definiert ist, oder Tetrazol, 5-(C₁-C₄-Alkylthio)-1H-tetrazol oder 5-(C₆-C₁₂)-Aryl-1H-tetrazol, oder anderen Aktivatoren wie beispielsweise Pyridin-Hydrochlorid, vorzugsweise in Gegenwart von Tetrazol oder Pyridin-Hydrochlorid, umsetzt
(f) die erhaltene Verbindung nach bekannten Verfahren oxidiert, beispielsweise durch Umsetzung mit Jod in Gegenwart von wäßrigem Pyridin, Lutidin oder Collidin, gegebenenfalls auch in Gegenwart weiterer organischer Lösungsmittel wie beispielsweise Tetrahydrofuran, oder beispielsweise durch Umsetzung mit tert-Butylhydroperoxid in Tetrahydrofuran, oder durch Umsetzung mit N,N,N',N'-Tetraethylthiuramdisulfid in Acetonitril, oder beispielsweise durch Umsetzung mit Jod in Gegenwart von Alkylamin oder Arylamin, wobei die verschiedenen, dem Fachmann bekannten Oxidationsverfahren, die der Herstellung von natürlichen und modifizierten Oligonucleotiden dienen, beispielsweise in Beaucage und Iyer [Tetrahedron 49 (1993) 6123] sowie Uhlmann und Peyman [Chem. Rev. 90 (1990) 543] zusammengefaßt sind, wobei die Oxidation bevorzugt durch Umsetzung mit Jod in Gegenwart von wäßrigem Pyridin, Lutidin oder Collidin, gegebenenfalls auch in Gegenwart weiterer organischer Lösungsmittel wie Tetrahydrofuran erfolgt;
(f)' gegebenenfalls nicht abreagierte Verbindungen aus Schritt (d) durch einen capping-Schritt deaktiviert, beispielsweise durch Umsetzung mit Acetanhydrid-Lutidin-N-Methylimidazol in THF;
(g) die Reaktionsschritte d - f bis zur gewünschten Kettenlänge wiederholt;
(h) die so erhaltene Verbindung entschützt durch Behandlung mit einer 0.1 bis 5 M Lösung von DBU in einem geeigneten organischen Lösungsmittel wie beispielsweise Acetonitril, Pyridin oder N-Methylimidazol bei 0 bis 70 °C während 0.1 bis 16h, bevorzugt mit einer 0.3 bis 3M Lösung bei 10 bis 40°C während 0.1 bis 2h, besonders bevorzugt mit einer 0.5 bis 2.5 M Lösung zwischen 20 und 30 °C während 0.2 bis 1.5 h;
(i) das Oligonucleotid nach bekannten Verfahren vom Träger abspaltet, beispielhaft mit NH₃ bei 20-30 °C und die Verbindungen der Formel I durch Lyophilisation der ammoniakalischen Lösung erhält.

Durch starke, nicht nukleophile Basen abspaltbare Schutzgruppen sind solche, die durch Behandlung mit starken, nicht nukleophilen Basen abgespalten werden, wobei die Abspaltung in der Regel durch β-Eliminierung erfolgt. Solche Schutzgruppen sind beispielsweise 4-Nitrophenylethyl, 2-Cyanoethyl, Dansylsulfonylethyl, Arylethyl, Arylsulfonylethyl, wobei Phenyl gegebenenfalls ein oder mehrfach substituiert mit Chlor, Brom, CN, NO₂, F, sein kann, bevorzugt 4-Nitrophenylethyl oder 2-Cyanoethyl.

Die Synthese der Verbindungen der Formel VII kann auch erfolgen durch Succinoylierung des festen Trägers und anschließende Aufkondensation von Verbindungen der Formel III.

Die Einführung der Gruppen Q und Q' erfolgt gegebenenfalls nach dem Fachmann bekannten Verfahren (s. z.B. Uhlmann & Peyman, Chem. Rev. 90 (1990) 543; M. Manoharan in "Antisense Research and Applications", Crooke and Lebleu, Eds., CRC Press, Boca Raton, 1993, Chapter 17, S.303ff. und EP-A 0 552 766).
Schutzgruppen für R^{1'}, wenn R¹ OH oder Amino darstellt und die Synthese entsprechend derivatisierter Nucleosidbausteine sind dem Fachmann bekannt (wie beispielsweise die Dimethyltertbutylsilylgruppe oder die 1-(2-chloro-4-methylphenyl)-4-methoxy-4-piperidinylgruppe für R¹ ist Hydroxy oder die Acetylgruppe für R¹ ist Amino) und sind beispielsweise beschrieben in Uhlmann und Peyman Chem. Rev. 90 (1990) 543; Beaucage und lyer Tetrahedron 48 (1993) 2223 oder C. Hendrix et al., Nucl Acids Res. 26 (1995) 51.

Beispiele für B^{PR} sind 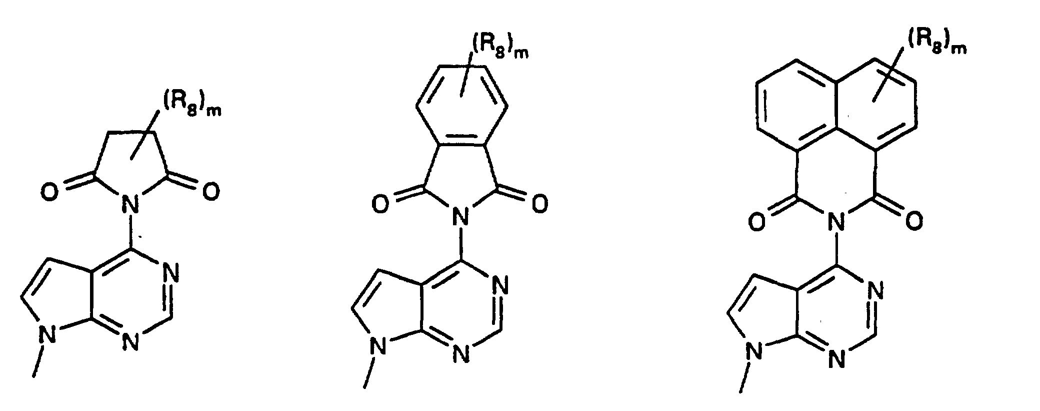 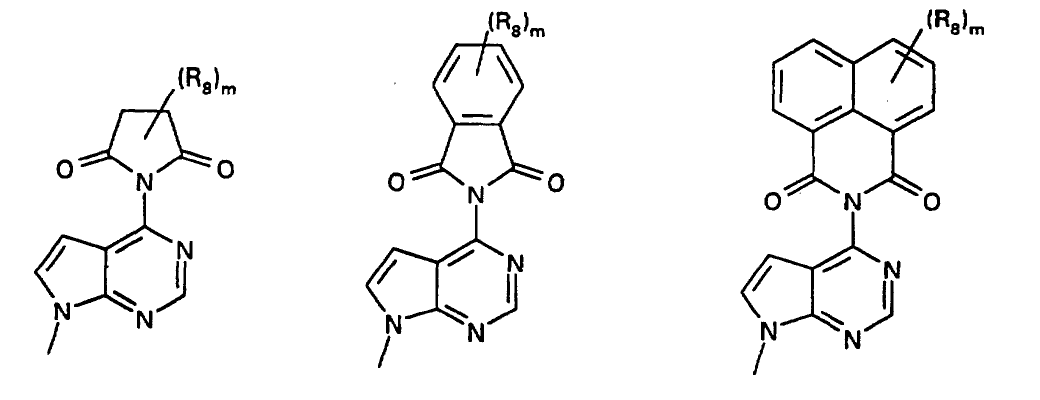 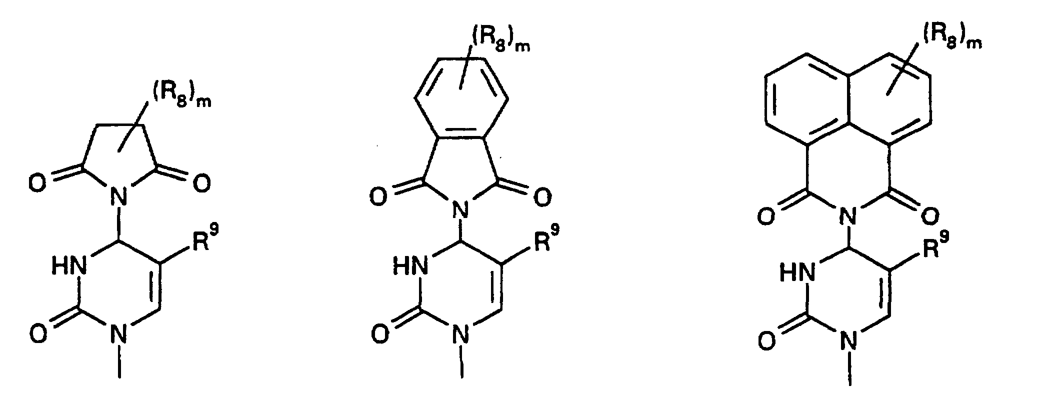 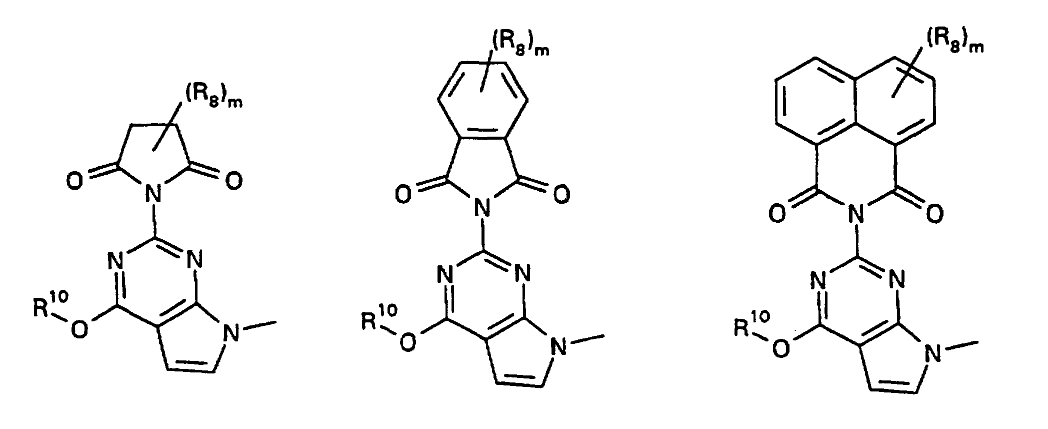 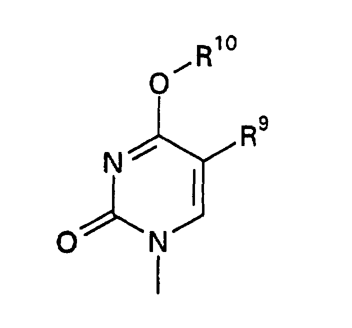 worin
- m: für eine Zahl von Null bis vier, bevorzugt für Null steht, die Reste
- R⁸: unabhängig voneinander für Wasserstoff, Fluor, Chlor, Brom, Nitro, C₁-C₄-Alkyl, C₁-C₄-Alkoxy, CN steht,
- R⁹: unabhängig voneinander Wasserstoff, C₁-C₆-Alkyl, C₂-C₆-(1-Alkin), bevorzugt 1-Propinyl und 1-Hexinyl oder Fluor bedeutet; und
- R¹⁰: unabhängig voneinander für Wasserstoff oder eine β-eliminierbare Schutzgruppe, wie beispielsweise para-Nitrophenylethyl, Phenylsulfonylethyl stehen,
wobei die Einführung cyclischer Diacyl-Schutzgruppen und der 5'-Schutzgruppe S in Nucleoside dem Fachmann bekannt ist, s. z.B. Kume et al., Tetrahedron Lett. 23 (1982) 4365; Nucleic Acids Res. 12 (1984) 8525; Nucleic Acids Res. Symp. Ser. 11 (1982) 26; Chemistry Letters 1983, 1597 oder Dikshit et al., Can. J. Chem. 66 (1988) 2989 oder Kamaike et al., Tetrahedron Lett. 36 (1995) 91.
Modifizierte Nucleosid-Bausteine lassen sich in analoger Weise schützen. Die Einführung der Schutzgruppen R¹⁰ erfolgt vor der Einführung der cyclischen Diacylgruppen ebenfalls nach bekannten Verfahren, beispielsweise nach F. Himmelsbach et al., Tetrahedron 40 (1984) 59 oder Beaucage und lyer Tetrahedron 49 (1993) 6123; Beaucage und lyer Tetrahedron 48 (1993) 2223.

Bevorzugt ist das erfindungsgemäße Verfahren zur Herstellung von Verbindung der Formel I, worin
- R¹: Wasserstoff, Hydroxy, C₁-C₄-Alkoxy oder Fluor bedeutet;
- A: für Oxy steht;
- W: Oxo oder Thioxo bedeutet;
- V: Oxy bedeutet;
- Y: Oxy bedeutet;
- B: unabhängig voneinander für Adenin, Cytosin, Guanin, Uracil, Thymin, 5-Propinuracil und 5-Propincytosin, 5-Hexinuracil oder 5-Hexincytosin steht, wobei mindestens ein B für eine Base steht, die eine exocyclische Aminogruppe aufweist,
- n: eine ganze Zahl von 5 bis 40 bedeutet;
- U: für für Hydroxy, Mercapto, C₁-C₆-Alkoxy, C₁-C₆-Alkyl, NR³R⁴ oder NHR³ steht, worin
R³ für C₁-C₈-Alkyl oder Methoxyethyl steht;
R⁴ C₁-C₈-Alkyl, C₆-C₂₀-Aryl oder (C₆-C₁₀)-Aryl-(C₁-C₈)-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann; und
- Q und Q': unabhängig voneinander Wasserstoff bedeuten.

Besonders bevorzugt ist das erfindungsgemäße Verfahren zur Herstellung von Verbindungen der Formel I, worin
- R¹: Wasserstoff bedeutet
- A: für Oxy steht;
- W: Oxo oder Thioxo bedeutet;
- V: Oxy bedeutet;
- Y: Oxy bedeutet;
- B: für Adenin, Cytosin, Guanin, Uracil, Thymin, 5-Propinuracil und 5-Propincytosin, 5-Hexinuracil oder 5-Hexincytosin steht;
wobei mindestens ein B für eine Base steht, die eine exocyclische Aminogruppe aufweist;
- n: bevorzugt 5 bis 30 bedeutet;
- u: Hydroxy oder C₁-C₆-Alkyl bedeutet, und
- Q und Q': Wasserstoff bedeuten.

Die nach dem erfindungsgemäßen Verfahren hergestellten Oligonukleotide sind vielfältig einsetzbar, z.B. als Inhibitoren der Genexpression, als Ribozyme, oder als Sonden in der Diagnostik (insbesondere als DNA-Sonden), oder ganz allgemein als Hilfsmittel in der Molekularbiologie.

Gegenstand der Erfindung ist weiterhin eine Verbindung der Formel V 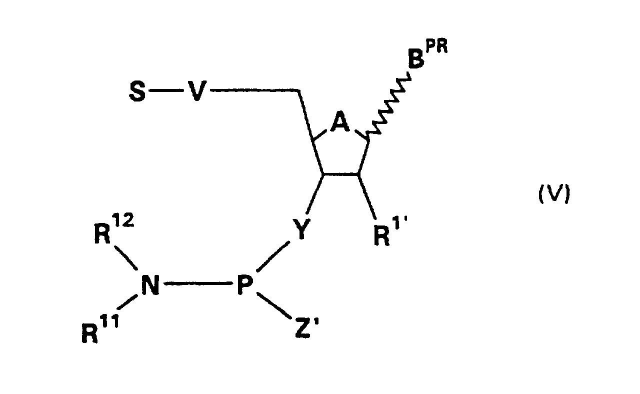 worin
- R^{1'}: unabhängig voneinander Wasserstoff, C₁-C₁₈-Alkoxy, gegebenenfalls ein bis dreifach substituiert mit Hydroxy, oder C₁-C₄-Alkoxy, C₁-C₄-Alkyl-O-(CH₂CH₂O)₁₋₃, O-Allyl, Fluor, Chlor, Azido oder eine geschützte Hydroxyoder Aminogruppe bedeutet;
- A: für Oxy, Thioxy oder Methylen steht;
- V: Oxy, Sulfandiyl oder Imino bedeutet;
- Y: Oxy, Sulfandiyl, Imino oder Methylen bedeutet;
S für eine im sauren abspaltbare 5'-Schutzgruppe wie beispielsweise Dimethoxytrityl, Monomethoxytrityl, Trityl, Pixyl, bevorzugt Dimethoxytrityl und Monomethoxytrityl steht;
B^{PR} für eine natürliche oder unnatürliche Nucleobase mit exocyclischer Aminogruppe steht, wobei die exocyclischen Aminogruppen durch eine cyclische Diacylgruppe geschützt ist;
Z' für OR¹³ oder C₁-C₁₈-Alkyl, C₁-C₁₈-Alkoxy, C₆-C₂₀-Aryl, C₆-C₁₄-Aryl-C₁-C₈-Alkyl, steht;
R¹¹ und R¹² gleich oder verschieden sind und C₁-C₈-Alkyl, vorzugsweise Isopropyl, oder C₅-C₁₂-Cycloalkyl, vorzugsweise bis C8, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-Alkyl-Ester, bedeuten; und
R¹³ für para-Nitrophenylethyl oder 2-Cyanoethyl steht.

Bevorzugt sind Verbindung der Formel V, worin
- B^{PR}: 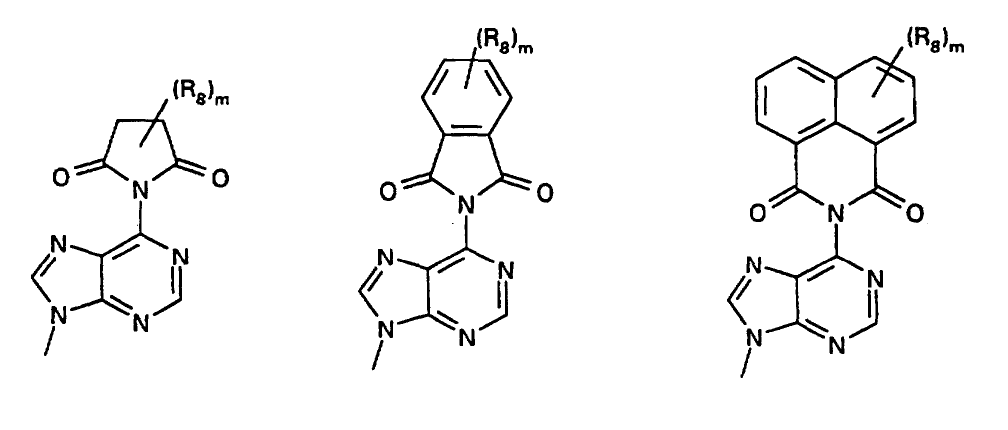 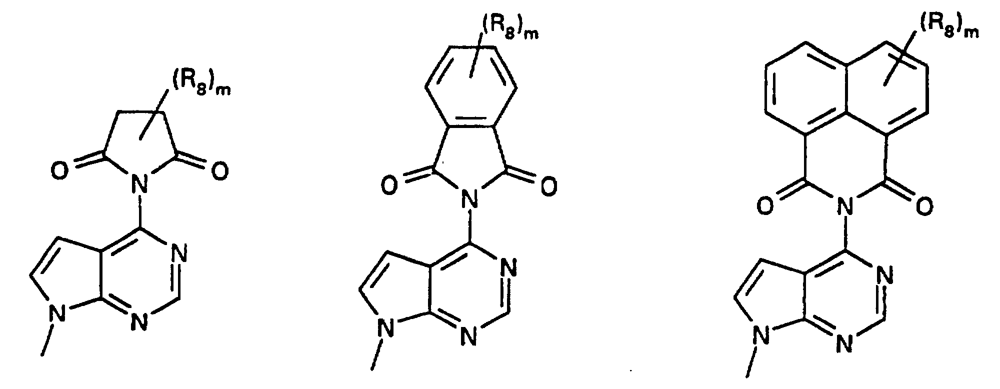 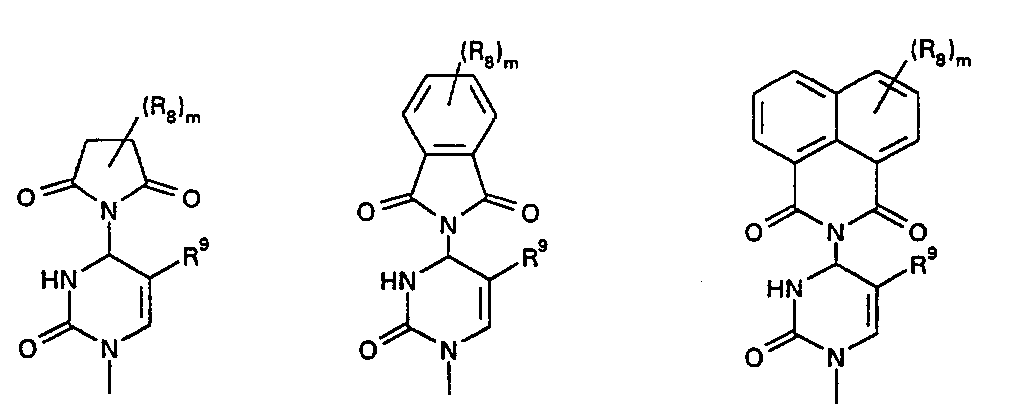 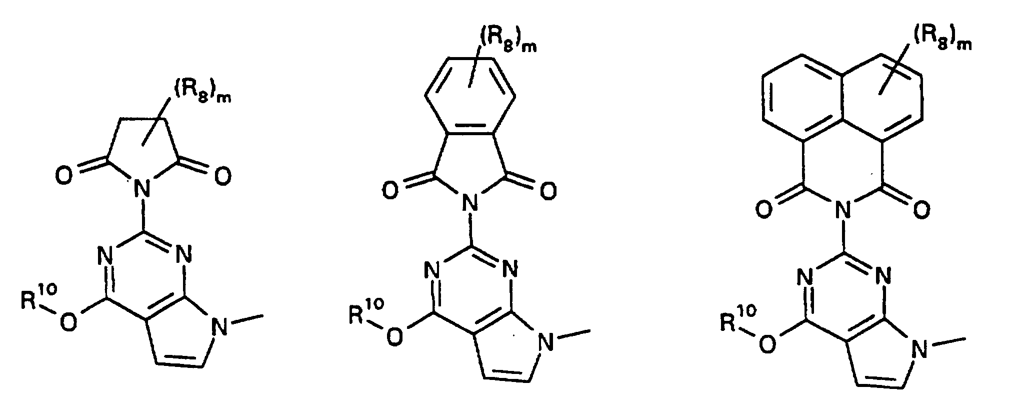 bedeutet,
worin
m für eine Zahl von Null bis vier, bevorzugt für Null steht; und
R⁸ unabhängig voneinander für Wasserstoff, Fluor, Chlor, Brom, Nitro, C₁-C₄-Alkyl, C₁-C₄-Alkoxy, CN steht;
und R⁹ und R¹⁰ wie oben definiert sind.

Besonders bevorzugt sind Verbindungen der Formel V, worin
- R^{1'}: Wasserstoff, eine geschützte Hydroxy-Gruppe, C₁-C₄-Alkoxy oder Fluor bedeutet,
- A: für Oxy steht;
- V: Oxy bedeutet;
- Y: Oxy bedeutet;
- Z': OR¹³, C₁-C₆-Alkyl, C₁-C₆-Alkoxy, C₆-C₂₀-Aryl, C₆-C₁₄-Aryl-C₁-C₈-Alkyl, bevorzugt OR¹³ bedeuten.

Die Verbindungen der Formel V sind wichtige Zwischenprodukte für die erfindungsgemäße Herstellung der Verbindungender Formel I.

### Beispiele:

### Beispiel 1

### 5'-O-Dimethoxytrityl-N⁶-phtaloyl-2'-O-desoxy-adenosin-3'-O-(N,N-diisopropyl-[2-(4-nitrophenyl]ethyl])-phosphitamid

### 1.1 N⁶-Phtaloyl-2'-O-desoxy-adenosin

5 g 2'-O-Desoxyadenosin (20 mmol) werden zunächst mit absolutem Pyridin koevaporiert, dann in 80 ml Pyridin gelöst und mit 6 ml Chlortrimethylsilan (50 mmol) versetzt und 30 min bei Raumtemperatur (RT) gerührt. Dann werden 4 ml Phthaloylchlorid (28 mmol) zugegeben. Nach 2 hr wird mit Eiswasser hydrolysiert und 10 min nachgerührt. Man verdünnt mit 150 ml AcOEt und extrahiert viermal mit 100 ml gesättigter Natriumchlorid-Lösung und die wässrige Phase viermal mit 50 ml AcOEt zurück. Zur Reinigung nimmt man in 50 ml Dichlormethan auf und fällt aus 1000 ml Petrolether aus. Man erhält 7.37 g (18.5 mmol ; 93 %) eines ocker gefärbten Feststoffs.

### 1.2 5'-O-Dimethoxytrityl-N⁶-phthaloyl-2'-O-desoxy-adenosin (Literatur : Akiko Kume, Mitsuo Sekine, Tsujiaki Hata, Tetrahedron Letters 23(42),4365-4368 (1982))

4 g N⁶-Phtaloyl-2'-O-desoxy-adenosin (10 mmol) werden mit absolutem Pyridin koevaporiert, dann in 50 ml absolutem Pyridin gelöst und 50 ml Dichlormethan mit 20 mg N,N-Dimethylaminopyridin (0.16 mmol) und Molsieb 4 Å werden 2.5 hr mit 2.72 g 4,4-Dimethoxytritylchlorid (8 mmol) gerührt. Nach Abziehen des Lösungsmittels wird in 100 ml Dichlormethan gelöst und mit 100 ml Natriumhydrogencarbonatlösung extrahiert. Die vereinigten organischen Phasen werden über Magnesiumsulfat getrocknet. Man filtriert ab, rotiert ein und koevaporiert mit Toluol. Es wird über Kieselgel (100 g) mit einem Toluol-AcOEt-Gradienten (44-80 % AcOEt) chromatographiert. Es ergeben sich 3.12 g (4.56 mmol 46 %) weisser Schaum.

### 1.3 5'-O-Dimethoxytrityl-N⁶-phthaloyl-2'-O-desoxy-adenosin-3'-O-(N,N-diisopropyl-[2-(4-nitrophenyl)ethyl])-phosphitamid

400 mg 5'-O-Dimethoxytrityl-N⁶-phthaloyl-2'-desoxyadenosin (0.58 mmol) werden in 5 ml Acetonitril gelöst, 313 mg Bis-(N,N-diisopropylamino)-2-(4-nitrophenyl)ethoxy-phosphan (0.79 mmol) und 20 mg Tetrazol (0.29 mmol) zugegeben. Nach 1 hr Rühren unter Schutzgas wird einrotiert, in 50 ml Dichlormethan gelöst und gegen 50 ml gesättigte Natriumhydrogencarbonat-Lösung extrahiert. Nach Tocknen über MgSO₄ wird einrotiert. Man chromatographiert über Kieselgel (6g SiO₂) mit Toluol-AcOEt (1:1) und erhält 380 mg (0.39 mmol ; 67 %) weissen Schaum.
DC (Kieselgel) : R_{f} = 0.79 (Toluol/AcOEt 1:9)); ¹H-NMR (DMSO, 250 MHz): 8.91 (s, 1H, H-C(2)), 8.80 (s, 1H, H-C(8)), 8.07 (m, 6H, 4H pth, 2 H o zu NO₂), 7.46 (d, 2H, 2H m zo NO₂), 7.16 (m, 9H, DMTr), 6.80 (m, 4H, o zu OCH₃), 6.51 (t, 1H, H-C(1')), 4.75 (m, 1H, H-C(3')), 4.13 (m, 1H, H-C(4')), 3.75 (m, 8H, CH₂O, 2x O-CH³), 3.50 (m, 2H, H-C(5'), H-C(5'')), 3.20 (m, 6H, CH₂-Phenyl, 2x N-CH, H-C(2'), H-C(2'')), 1.01 (m, 12 H, 2x C(CH₃)₂); ³¹P-NMR (DMSO, 161.7 MHz) :1s 147.50 ; C₅₃H₅₄N₇O₁₀P₁ (980.04) ; ber.: C 64.95, H 5.55, N 10.00 ; gef.: C 64.15, H 5.53, N 9.72

### Beispiel 2:

### 5'-O-Dimethoxytrityl-N⁶-phthaloyl-2'-O-desoxy-adenosin-3'-O-(β-cyanoethyl-N,N-diisopropyl)-phosphitamid

500 mg 5'-O-Dimethoxytrityl-N⁶-phthaloyl-2'-desoxyadenosin(Beispiel 1.2) (0.73 mmol) werden in 5 ml Acetonitril gelöst, 280 mg Bis-(N,N-diisopropylamino)-2-cyanoethoxy-phosphan (0.92 mmol) und 26 mg Tetrazol (0.37 mmol) zugegeben. Nach 1.5 hr Rühren unter Schutzgas wird mit 70 ml Dichlormethan und 60 ml Natriumhydrogencarbonat-Lösung extrahiert. Nach Tocknen über MgSO₄ wird einrotiert. Man chromatographiert über Kieselgel (9g SiO₂) in einem Toluol-AcOEt-Gradienten (40-50 % AcOEt) und erhält 430 mg (0.49 mmol ; 67 %) weissen Schaum.
DC (Kieselgel) : R_{f} = 0.33/0.42 42 (Toluol/AcOEt 1:1)); ¹H-NMR (DMSO, 250 MHz):
8.92 (s, 1H, H-C(2)), 8.82 (s, 1H, H-C(8)), 8.09 (m, 4H, pth), 7.33 (m, 2H, DMTr), 7.21 (m, 7H, DMTr), 6.82 (m, 4H, o zu OCH₃), 6.55 (m, 1H, H-C(1')), 4.82 (m, 1H, H-C(3')), 4.27 (m, 1H, H-C(4')), 3.65 (m, 10H, CH₂O, 2x O-CH₃, H-C(5'), H-C(5'')), 3.20 (m, 4H, 2x N-CH, H-C(2'), H-C(2'')), 1.10 (m, 12 H, 2x C(CH₃)₂); ³¹P-NMR (DMSO, 161.7 MHz) : 2s 148.51/147.99 ; C₄₈H₅₀N₇O₈P₁ (883.95) ; ber.: C 65.22, H 5.70, N 11.09 ; gef.: C 64.94, H 5.78, N 10.87

### Beispiel 3:

### 5'-O-Dimethoxytrityl-N⁶-phthaloyl-2'-O-desoxy-cytidin-3'-O-(β-cyanoethyl-N,N-diisopropyl)-phosphitamid

### 3.1 N⁶-Phthaloyl-2'-O-desoxy-cytidin

Die Synthese erfolgt analog Beispiel 1.1: 5.8 g 2'-O-Desoxy-cytidin (22 mmol) werden in 80 ml Pyridin mit 7 ml Chlortrimethylsilan (55 mmol) gerührt und nach 1 hr innerhalb 60 min 4.4 ml Phthaloylchlorid (33 mmol) in 10 ml trockenem Dioxan zugetropft. Es werden 50 mg DMAP zugegeben, nach 45 min mit Wasser abgestoppt und 15 min nachgerührt. Extrahiert wird mit 10 % Pyridin in Dichlormethan gegen 100 ml Wasser . Die organische Phase wird mit 100 ml Wasser gewaschen und die wässrige Phase zweimal mit je 50 ml 10 % Pyridin in Dichlormethan rückextrahiert. Nach Trocknen, Abrotieren und Koevaporation mit Toluol wird in Dichlormethan aufgeschlämmt und abgesaugt. Es ergeben sich 5.07 g (14.2 mmol ; 64 %) eines ocker gefärbten Pulvers.
DC (Kieselgel) : R_{f} = 0.10 (Toluol/AcOEt/MeOH 5:4:1)); ¹H-NMR (DMSO, 250 MHz): 8.63 (d, 1H, H-C(6)), 8.01 (m, 4H, pth), 6.69 (d, 1H, H-C(5)), 6.10 (t, 1H, H-C(1')), 5.30 (d, 1H, HO-C(3')), 5.11 (t, 1H, HO-C(5')), 4.23 (m, 1H, H-C(3')), 3.91 (m, 1H, H-C(4')), 3.63 (m, 2H, H-C(5'), H-C(5'')), 2.38 (m, 1H, H-C(2')), 2.14 (m, 1H, H-C(2")); UV (ACN): λmax (nm) / log ε: [331/3.68], 316/3.80, [306/3.79], 218/4.51; C₁₇H₁₅N₃O₆ (357.32) ; ber.: C 57.14, H 4.23, N 11.76 ; gef.: C 57.21, H 4.45, N 11.66

### 3.2 5'-O-Dimethoxytrityl-N⁶-phthaloyl-2'-O-desoxy-cytidin

Die Synthese erfolgt analog Beispiel 1.2: 3 g N⁶-phthaloyl-2'-O-desoxy-cytidin (8.39 mmol) in 60 ml Pyridin mit 50 mg N,N-Dimethylaminopyridin (0.41 mmol) und Molsieb 4 Å werden 2 hr mit 3.13 g 4,4-Dimethoxytritylchlorid (9.23 mmol) gerührt. Nach Extraktion mit 100 ml Dichlormethan und 100 ml Natriumhydrogencarbonatlösung wird über Kieselgel (75 g) mit einem Toluol-AcOEt-Gradienten (33-75 % AcOEt) chromatographiert. Es ergeben sich 3.35 g (5.10 mmol ; 61 %) weisser Schaum.
DC (Kieselgel) : R_{f} = 0.39 (Toluol/AcOEt/MeOH 5:4:1)); ¹H-HMR (DMSO, 250 MHz): 8.42 (d, 1H, H-C(6)), 7.99 (m, 4H, pth), 7.30 (m, 9H, DMTr), 6.92 (m, 4H, o zu OCH₃), 6.53 (d, 1H, H-C(5)), 6.11 (t, 1H, H-C(1')), 5.40 (d, 1H, HO-C(3')), 4.31 (m, 1H, H-C(3')), 4.01 (m, 1H, H-C(4')), 3.73 (s, 6H, 2x O-CH₃), 3.33 (m, 2H, H-C(5'), H-C(5'')), 2.40 (m, 1H, H-C(2')), 2.20 (m, 1H, H-C(2'')); UV (ACN): λmax (nm) / log ε: [331/3.68], 317/3.84, [306/3.83], 283/3.72, [275/3.69], 232/4.65; C₃₈H₃₃N₃O₈ (659.70) ; ber.: C 69.19, H 5.04, N 6.37 ; gef.: C 69.29, H 5.35, N 6.17

### 3.3 5'-O-Dimethoxytrityl-N⁶-phthaloyl-2'-O-desoxy-cytidin-3'-O-(β-cyanoethyl-N,N-diisopropyl)-phosphitamid

Die Synthese erfolgt analog Beispiel 2: 660 mg 5'-O-Dimethoxytrityl-N⁶phthaloyl-2'-desoxycytidin (1 mmol) werden in 7 ml Acetonitril gelöst, 362 mg Bis-(N,N-diisopropylamino)-2-cyanoethoxy-phosphan (1.2 mmol) und 35 mg Tetrazol (0.5 mmol) zugegeben. Nach 1.5 hr wird mit 100 ml Dichlormethan und 60 ml Natriumhydrogencarbonat-Lösung extrahiert. Man chromatographiert über Kieselgel (8g SiO₂) in einem Toluol-AcOEt-Gradienten (40-50 % AcOEt) und erhält 620 mg (0.72 mmol ; 72 %) weissen Schaum.
DC (Kieselgel) : R_{f} = 0.47/0.51 (Toluol/AcOEt 1:6)); ¹H-NMR (DMSO, 250 MHz):
8.45 (m, 1H, H-C(6)), 7.99 (m, 4H, pth), 7.30 (m, 9H, DMTr), 6.91 (m, 4H, o zu OCH₃), 6.55 (d, 1H, H-C(5)), 6.15 (m, 1H, H-C(1')), 4.52 (m, 1H, H-C(3')), 4.17 (m, 1H, H-C(4')), 3.72 (s, 6H, 2x O-CH₃), 3.60 (m, 6H, H-C(5'), H-C(5"), CH₂O, 2x N-CH)), 2.70 (2t, 2H, CH₂CN), 2.60 (m, 1H, H-C(2')), 2.40 (m, 1H, H-C(2'')), 0.97-1.19 (m, 12 H, 2x C(CH₃)₂)
³¹P-NMR (DMSO, 161.7 MHz) : 2s 148.63/148.38 ; C₄₇H₅₀N₅O₈P₁ (859.91); ber.: C 65.65, H 5.86, N 8.14 ; gef.: C 64.16, H 6.04, N 8.23

### Beispiel 4:

### 5'-O-Dimethoxytrityl-O⁶-[2-(4-nitrophenyl)ethyl]-N²-phthaloyl-2'desoxyguanosin-3'-O-(cyanoethyl-N,N-diisopropyl)-phosphitamid

### 4.1 O⁶-[2-(4-Nitrophenyl)ethyl]-N²-phthaloyl-2'-desoxyguanosin

Die Synthese erfolgt analog Beispiel 1.1

### Variante A : Abspaltung der Trimethylsilyl-Gruppen mit Pyridin-Wasser

833 mg 06-[2-(4-Nitrophenyl)ethyl]-2'-desoxyguanosin (2 mmol) werden mit 0.63 ml Chlortrimethylsilan (5 mmol) in 15 ml Pyridin gerührt und nach 30 min werden 0.43 ml Phthaloylchlorid (3 mmol) in 5 ml trockenem Dioxan innerhalb 15 min zugetropft. Nach 2 hr wird mit Wasser abgestoppt und 15 min nachgerührt. Extrahiert wird zweimal mit 50 ml Natriumhydrogencarbonat-Lösung gegen 50 ml Dichlormethan sowie die wässrigen Phasen nochmals mit 50 ml Dichlormethan. Man trocknet über MgSO4, filtriert und engt ein.Die Aufreinigung erfolgt über eine Kieselgelsäule (25 g SiO₂) mittels Flash-Chromatographie in einem Toluol/AcOEt (5:4)-MeOH-Gradienten (0-5 % MeOH). Man erhält 0.56 g (1.02 mmol ; 51 %) eines leicht gelblich gefärbten Schaums.

### Variante B : Abspaltung der Trimethylsilyl-Gruppen mit Ammoniumfluorid

833 mg O⁶-[2-(4-Nitrophenyl)ethyl]-2'-desoxyguanosin (2 mmol) werden mit 0.63 ml Chlortrimethylsilan (5 mmol) in 15 ml Pyridin gerührt und nach 30 min werden 0.57 ml Phthaloylchlorid (4 mmol) in 2 ml trockenem Dioxan innerhalb 5 min zugetropft. Nach 2 hr wird einrotiert, mit Toluol koevaporiert und mit 160 mg Ammoniumfluorid (4.3 mmol) in 20 ml MeOH versetzt. Man rührt 3 min, extrahiert zweimal mit 100 ml Natriumhydrogencarbonat-Lösung gegen 100 ml Dichlormethan und die wässrigen Phasen nochmals mit 50 ml Dichlormethan. Die Aufreinigung erfolgt über eine Kieselgelsäule (25 g SiO₂) mittels Flash-Chromatographie in einem Toluol/AcOEt (5:4)-MeOH-Gradienten (0-5 % MeOH). Man erhält 0.45 g (0.82 mmol ; 41 %) eines leicht gelblich gefärbten Schaums.

### Variante C : Abspaltung der Trimethylsilyl-Gruppen mit Tetrabutylammoniumfluorid

833 mg O⁶-[2-(4-Nitrophenyl)ethyl]-2'-desoxyguanosin (2 mmol) werden mit 0.7 ml Chlortrimethylsilan (5.5 mmol) in 15 ml Pyridin gerührt und nach 30 min werden 0.55 ml Phthaloylchlorid (3.8 mmol) in 3 ml trockenem Dioxan innerhalb 10 min zugetropft. Nach 2 hr werden 1.26 g TetrabutylammoniumfluoridTrihydrat (4 mmol) zugesetzt und 5 min gerührt. Anschließend extrahiert man zweimal mit 100 ml Natriumhydrogencarbonat-Lösung gegen 100 ml Dichlormethan und die wässrigen Phasen nochmals mit 50 ml Dichlormethan. Die Aufreinigung erfolgt über eine Kieselgelsäule (25 g SiO₂) mittels Flash-Chromatographie in einem Toluol/AcOEt (5:4)-MeOH-Gradienten (0-5 % MeOH). Man erhält 0.39 g (0.71 mmol ; 36 %) eines leicht gelblich gefärbten Schaums.
DC (Kieselgel) : R_{f} = 0.42 (Chloroform/Methanol 9:1); ¹H-NMR (DMSO, 250 MHz): 8.71 (s, 1H, H-C(8)), 8.14 (d, 2H, 2H o zu NO₂), 8.00 (m, 4H, pth), 7.61 (d, 2H, 2H m zu NO₂), 6.39 (t, 1H, H-C(1')), 5.32 (d, 1H, HO-C(3')), 4.91 (t, 1H, HO-C(5')), 4.83 (t, 2H, CH₂O), 4.40 (m, 1H, H-C(3')), 3.85 (m, 1H, H-C(4')), 3.51-3.58 (m, 2H, H-C(5'), H-C(5")), 3.32 (t, 2H, CH₂-Phenyl), 2.74 (m, 1H, H-C(2')), 2.32 (m, 1H, H-C(2'')); UV (ACN): λmax (nm) / log e: 262/4.34, 219/4.68; C₂₆H₂₂N₆O8 (546.50) ; ber.: C 57.14, H 4.06, N 15.38 ; gef.: C 57.27, H 4.37, N 15.11

### 4.2 5'-O-Dimethoxytrityl-O⁶-[2-(4-nitrophenyl)ethyl]-N²-phthaloyl-2'desoxyguanosin

Synthese analog Beispiel 1.2: 990 mg O⁶-[2-(4-Nitrophenyl)ethyl]-N²-phthaloyl-2'-desoxyguanosin (1.81 mmol) werden in 25 ml Pyridin gelöst, 674 mg 4,4-Dimethoxytritylchlorid (1.99 mmol) zugegeben und für 3 hr gerührt. Man extrahiert mit 100 ml AcOEt gegen 100 ml gesättigte Natriumhydrogencarbonatlösung. Anschließend chromatographiert man über Kieselgel (25 g SiO₂) in einem Toluol-AcOEt-Gradienten (20-66 % AcOEt) und erhält 1.00 g (1.18 mmol ; 67 %) weissen Schaum.
DC (Kieselgel) : R_{f} = 0.57 (Toluol/AcOEt 1:6); ¹H-NMR (DMSO, 250 MHz): 8.59 (s, 1H, H-C(8)), 8.13 (d, 2H, 2H o zu NO₂), 8.00 (m, 4H, pth), 7.60 (d, 2H, 2H m zu NO₂), 7.10-7.26 (m, 9H, DMTr), 6.68 (m, 4H, o zu OCH₃), 6.43 (m, 1H, H-C(1')), 5.36 (d, 1H, HO-C(3')), 4.81 (t, 2H, CH₂O), 4.46 (m, 1H, H-C(3')), 3.95 (m, 1H, H-C(4')), 3.67 (s, 6H, 2x O-CH₃), 3.32 (t, 2H, CH₂-Phenyl), 3.05-3.25 (m, 2H, H-C(5'), H-C(5")), 2.85 (m, 1H, H-C(2')), 2.38 (m, 1H, H-C(2'')); UV (ACN): λmax (nm) / log ε: 262/4.38, 218/4.78 C₄₇H₄₀N₆O₁₀ (848.87) ; ber.: C 66.50, H 4.75, N 9.90 ; gef.: C 66.65, H 4.90, N 9.52

### 4.3 5'-O-Dimethoxytrityl-O⁶-[2-(4-nitrophenyl)ethyl]-N²-phthaloyl-2'desoxyguanosin-3'-O-(β-cyanoethyl-N,N-diisopropyl)-phosphitamid

Synthese analog Beispiel 1.3, jedoch unter Verwendung von Pyridin-Hydrochlorid anstelle von Tetrazol; Ausbeute: 87%.
DC (Kieselgel) : R_{f} = 0.74/0.82 (Toluol/AcOEt 1:6)); ¹H-HMR (DMSO, 250 MHz): 8.63 (s, 1H, H-C(8)), 8.13 (s, 2H, 2H o zu NO₂), 8.00 (m, 4H, pth), 7.61 (s, 2H, 2H m zu NO₂), 7.08-7.24 (m, 9H, DMTr), 6.63-6.71 (m, 4H, DMTr), 6.45 (m, 1H, H-C(1')), 4.80 (m, 3H, H-C(3'), CH₂O), 4.10 (m, 1H, H-C(4')), 3.66 u. 3.67 (2s, 6H, 2x OCH₃), 2.95-3.59 (m, 10 H, NC-CH₂O, H-C(5'), H-C(5''), 2x N-CH, CH₂CN, CH₂-Phenyl)2.72 (m, 1H, H-C(2')), 2.61 (m, 1H, H-C(2'')), 0.90-1.20 (m, 12 H, 2x C(CH₃)₂); ³¹P-NMR (DMSO, 161.7 MHz) : 2s 148.55/148.05 ; C₅₆N₅₇N₈O₁₁P₁ (1049.09) ; ber.: C 64.11, H 5.48, N 10.68 ; gef.: C 63.92, N 5.47, H 10.12

### Beispiel 5:

### 5'-O-Dimethoxytrityl-O⁶-[2-(phenylsulfonyl)ethyl]-N²-phthaloyl-2'desoxyguanosin-3'-O-(cyanoethyl-N,N-diisopropyl)-phosphitamid

### 5.1 O⁶-[2-(Phenylsulfonyl)ethyl]-N²-phthaloyl-2'-desoxyguanosin

Die Synthese erfolgt analog Beispiel 1.1

### Variante A : Abspaltung der Trimethylsilyl-Gruppen mit Pyridin-Wasser

300 mg O⁶-[2-(Phenylsulfonyl)ethyl]-2'-desoxyguanosin (0.69 mmol) werden mit 0.22 ml Chlortrimethylsilan (1.7 mmol) in 10 ml Pyridin gerührt und nach 30 min 0.14 ml Phthaloylchlorid (0.97 mmol) in 2 ml trockenem Dioxan innerhalb 7 min zugetropft. Nach 1 hr wird mit Wasser abgestoppt und 15 min nachgerührt. Extrahiert wird zweimal mit 25 ml Wasser gegen 25 ml Dichlormethan sowie die wässrigen Phasen nochmals mit 25 ml Dichlormethan. Die Aufreinigung erfolgt über eine Kieselgelsäule (7 g SiO₂) mittels Flash-Chromatographie in einem Petrolether-Aceton-Gradienten (50-66 % Aceton). Man erhält 0.14 g (0.24 mmol ; 36 %) eines nahezu farblosen Schaums.

### Variante B : Abspaltung der Trimethylsilyl-Gruppen mit Ammoniumfluorid

0.87 g O⁶-[2-(Phenylsulfonyl)ethyl]-2'-desoxyguanosin (2 mmol) werden mit 0.56 ml Chlortrimethylsilan (4.4 mmol) in 15 ml trockenem Acetonitril und 1.03 ml Pyridin gerührt und nach 30 min 0.29 ml Phthaloylchlorid (2 mmol) zugetropft. Nach 35 min einrotiert, mit Toluol koevaporiert und mit 160 mg Ammoniumfluorid (4.3 mmol) in 25 ml MeOH versetzt. Man rührt 3 min, extrahiert zweimal mit 100 ml Natriumhydrogencarbonat-Lösung gegen 100 ml Dichlormethan und die wässrigen Phasen nochmals mit 50 ml Dichlormethan. Die Aufreinigung erfolgt über eine Kieselgelsäule (25 g SiO₂) mittels Flash-Chromatographie in einem Petrolether-Aceton-Gradienten (30-75 % Aceton). Man erhält 0.45 g (0.79 mmol ; 40 %) eines leicht gelblich gefärbten Schaums.
DC (Kieselgel) : R_{f} = 0.27 (Petrolether/Aceton 1:2); ¹H-NMR (DMSO, 250 MHz): 8.65 (s, 1H, H-C(8)), 7.96-8.7 (m, 4H, pth), 7.82 (m, 2H, o zu SO₂), 7.36-7.49 (m, 3H, 2H m zu SO₂, 1H p zu SO₂), 6.37 (t, 1H, H-C(1')), 5.32 (d, 1H, HO-C(3')), 4.92 (t, 1H, HO-C(5')), 4.80 (t, 2H, CH₂O), 4.41 (m, 1H, H-C(3')), 4.12 (t, 2H, CH₂SO₂), 3.86 (m, 1H, H-C(4')), 3.45-3.65 (m, 2H, H-C(5'), H-C(5'')), 2.72 (m, 1H, H-C(2')), 2.33 (m, 1H, H-C(2'')); UV (ACN): λmax (nm) / log ε: [294/3.50] , 259/4.26 , 219/4.73; C₂₆H₂₃N₅O₈S₁ x H₂O (583,58) ber.: C 53,51; H 4,32; N 12,00; gef.: C 53,95; H 4,45; N 11,72

### 5.2 5'-O-Dimethoxytrityl-O⁶-[2-(phenylsulfonyl)ethyl]-N²-phthaloyl-2'desoxyguanosin

Synthese analog Beispiel 1.2: 560 mg O⁶-[2-(Phenylsulfonyl)ethyl]-N⁶phthaloyl-2'-desoxyguanosin (1 mmol) in 20 ml Pyridin werden mit 373 mg 4,4-Dimethoxytritiylchlorid (1.1 mmol) 16 hr gerührt. Man extrahiert mit 100 ml Dichlormethan gegen 100 ml gesättigte Natriumhydrogencarbonatlösung. Anschließend chromatographiert man über Kieselgel (20 g SiO₂) in einem Toluol-AcOEt-Gradienten (30-80 % AcOEt) und erhält 570 mg (0.66 mmol ; 66 %) leicht gelblichen Schaum.
DC (Kieselgel) : R_{f} = 0.39 (Toluol/AcOEt/MeOH 5:4:1); ¹H-NMR (DMSO, 250 MHz): 8.55 (s, 1H, H-C(8)), 8.01-8.07 (m, 4H, pth), 7.82 (m, 2H, 2H o zu SO₂), 7.32-7.38 (m, 3H, 2H m zu SO₂, 1H p zu SO₂ ), 7.05-7.27 (m, 9H, DMTr), 6.67-6.73 (m, 4H, o zu OCH₃), 6.41 (m, 1H, H-C(1')), 5.37 (d, 1H, HO-C(3')), 4.79 (t, 2H, CH₂O), 4.46 (m, 1H, H-C(3')), 4.10 (t, 2H, CH₂SO₂), 3.96 (m, 1H, H-C(4')), 3.68 (s, 6H, 2x OCH₃), 3.09-3.25 (m, 2H, H-C(5'), H-C(5'')), 2.81 (m, 1H, H-C(2')), 2.35 (m, 1H, H-C(2")); UV (ACN): λmax (nm) / log e: 261/4.26, 217/4.80; C₄₇H₄₁N₅O₁OS₁ (867.93) ; ber.: C 65.04, H 4.76, N 8.07 ; gef.: C 64.97, H 4.82, N 7.88

### 5.3 5'-O-Dimethoxytrityl-O⁶-[2-(phenylsulfonyl)ethyl]-N²-phthaloyl-2'desoxyguanosin-3'-O-(cyanoethyl-N,N-diisopropyl)-phosphitamid

Synthese analog Beispiel 2: 470 mg 5'-O-Dimethoxytrityl-O⁶-[2-(phenylsulfonyl)ethyl]-N²-phthaloyl-2'-desoxyguanosin (0.54 mmol) werden in 8 ml Acetonitril gelöst, 196 mg Bis-(N,N-diisopropylamino)-2-cyanoethoxyphosphan (0.65 mmol) und 0.54 ml einer 0.5 M Pyridiniumchlorid-Lösung zugegeben. Nach 3 hr werden weitere 150 mg Phosphan und 0.27 ml Pyridiniumchlorid-Lösung zugesetzt und für weitere 3.5 hr gerührt. Es wird mit 50 ml Dichormethan und 50 ml Natriumhydrogencarbonat-Lösung extrahiert. Man chromatographiert in einem Toluol-Esiigsäureethylester-Gradienten (33-50 % AcOEt) und erhält 420 mg (0.39 mmol ; 73 %) weissen Schaum.
DC (Kieselgel) : R_{f} = 0.49/0.60 (Petrolether/AcOEt/Triethylamin 1:9:1)); ¹H-NMR (DMSO, 250 MHz): 8.57 (2s, 1H, H-C(8)), 7.98-8.03 (m, 4H, pth), 7.81 (m, 2H, 2H o zu SO₂), 7.31-7.40 (m, 3H, 2H m zu SO₂, 1H o zu SO₂), 7.08-7.25 (m, 9H, DMTr), 6.39-6.73 (m, 4H, DMTr), 6.43 (m, 1H, H-C(1')), 4.78 (m, 3H, H-C(3'), CH₂O), 4.10 (m, 3H, CH₂SO₂, H-C(4')), 3.66 u. 3.67 (2s, 6H, 2x OCH₃), 3.30-3.60 (m, 4 H, NC-CH₂O, 2x N-CH), 3.23 (m, 2H, H-C(5'), H-C(5'')), 3.00 (m, 1H, H-C(2')), 2.62 u. 2.73 (2t, 2H, CH₂CN), 2.50 (m, 1H, H-C(2'')), 0.86-1.19 (m, 12 H, 2x C(CH₃)₂); ³¹P-NMR (DMSO, 161.7 MHz) : 2s 148.54/148.06 ; C₅₆H₅₈N₇O₁₁P₁S₁ (1068.15) ; ber.: C 62.97, H 5.47, N 9.18; gef.: C 62.25, N 5.65, H 8.82

### Beispiel 6:

### 5'-O-Dimethoxytrityl-N⁶-phtaloyl-3'-O-succinoyl-2'-O-desoxy-adenosin

342 mg 5'-O-Dimethoxytrityl-N⁶-phthaloyl-2'-O-desoxy-adenosin (Beispiel 1.2) (0.5 mmol) werden in 10 ml absolutem Dichlormethan gelöst und 79 mg 4,4-Dimethylaminopyridin (0.65 mmol) und 100 mg Bernsteinsäureanhydrid (1 mmol) zugesetzt. Nach 17 hr mit 50 ml Dichlormethan verdünnt und mit 30 ml gesättigter Natriumhydrogencarbonat-Lösung und dann mit 10 % Zitronensäure extrahiert. Nach Trocknen über MgSO₄, Abrotieren und Trocknen am Hochvakuum erhält man 390 mg (0.49 mmol ; 98 %) weissen Schaum.
DC (Kieselgel) : R_{f} = 0.17 (Toluol/AcOEt/MeOH 5:4:1)); ¹H-NMR (DMSO, 250 MHz):
12.30 (s, 1H, COOH), 8.91 (s, 1H, H-C(2)), 8.82 (s, 1H, H-C(8)), 8.06 (m, 4H, pth), 7.17 (m, 9H, DMTr), 6.83 (m, 4H, o zu OCH₃), 6.56 (t, 1H, H-C(1')), 5.45 (m, 1H, H-C(3')), 4.25 (m, 1H, H-C(4')), 3.70 (s, 6H, 2x O-CH₃), 3.33 (m, 2H, H-C(5'), H-C(5'')), 2.58 (m, 6H, H-C(2'), H-C(2"), CH₂CH₂); UV (ACN): λmax (nm) / log ε: [300/3.60], 271/4.17, [220/4.72]; C₄₃H₃₇N₅O₁₀ x H₂O (801.82) ; ber.: C 64.41, H 4.90, N 8.73 ; gef.: C 64.47, H 4.98, N 8.74

### Beispiel 7:

### 5'-O-Dimethoxytrityl-O⁶-[2-(4-nitrophenyl)ethyl]-N²-phthaloyl-3'-O-succinoyl-2'-O-desoxyguanosin

212 mg 5'-O-Dimethoxytrityl-O⁶-[2-(4-nitrophenyl)ethyl]-N²-phthaloyl-2'desoxyguanosin (0.25 mmol) werden in 5 ml absolutem Dichlormethan gelöst und 50 mg Bernsteinsäureanhydrid (0.5 mmol)und 40 mg 4,4-Dimethylaminopyridin (0.32 mmol) zugegeben. Nach 24 hr Rühren wird mit 60 ml Dichlormethan verdünnt und mit 40 ml gesättigter
Natriumhydrogencarbonat-Lösung und dann mit 40 ml 10 % Zitronensäurelösung extrahiert. Nach Trocknen über Magnesiumsulfat wird einrotiert und am HV getrocknet. Man erhält 210 mg (0.22 mmol ; 88 %) leicht gelblich gefärbten Schaum.
¹H-NMR (DMSO, 250 MHz): 12.27 (s, 1H, COOH), 8.61 (s, 1H, H-C(8)), 8.14 (d, 2H, 2H o zu NO₂), 7.96-8.05 (m, 4H, pth), 7.61 (d, 2H, 2H m zu NO₂),7.05-7.27 (m, 9H, DMTr), 6.65-6.71 (m, 4H, o zu OCH₃), 6.44 (t, 1H, H-C(1')), 5.36 (m, 1H, H-C(3')), 4.82 (t, 2H, OCH₂), 4.14 (m, 1H, H-C(4')), 3.67 (s, 6H, 2x O-CH₃), 3.16-3.42 (m, 4H, H-C(5'), H-C(5''), CH₂-Phenyl), 2.45-2.60 (m, 6H, H-C(2'), H-C(2"), CH₂CH₂); UV (ACN): λmax (nm) / log ε: 262/4.37 , 216/4.78.

### Beispiel 8:

### 8.1 Träger-Derivatisierung von Long Chain Methyl Amino Alkyl (LCMAA) -CPG :

1g CPG-Material 1000 Å oder 1400 Å werden 1.5 Stunden im HV getrocknet. 1 g Carbonyldiimidazol (12.4 mmol) wird in 20 ml trockenem Dichlormethan gelöst und das getrocknete CPG-Trägermaterial zugegeben und 6 Stunden am Rüttler gut durchmischt. Man dekantiert die überstehende Lösung ab und digeriert dreimal mit trockenem Dichlormethan. Anschließend wird in 15 ml trockenem Dichlormethan aufgenommen und mit 1 ml 1,6-Bis(methylamino)-hexan (5.8 mmol) versetzt. Nach 3 Stunden am Rüttler wird der Überstand abdekantiert, abgesaugt und nacheinander mit Pyridin, DMF, Methanol, Aceton und Diethylether gewaschen. Anschließend wird am HV getrocknet.

### 8.2 Beladung von LCAMAA-CPG mit Phthaloyl-geschützten Succinaten :

400 mg LCMAA-CPG werden mit 7 mg TOTU (O-[(ethoxycarbonyl)cyanomethylenamino]-N,N,N',N'-tetramethyluronium tetrafluoro-borate: W. König, G. Breipohl, P. Pokorny, M. Birkner, Proceedings of the 21st European Peptide Symposium 1990, E. Giralt, D. Andreu, Eds., ESCOM, Leiden, S. 143) .(21 µmol), 3 µl N-Methylmorpholin (27 µmol) und 23 µmol Nucleosidsuccinat für 2 Stunden in 5 ml trockenem Acetonitril geschüttelt. Man saugt ab und wäscht nacheinander mit DMF, Methanol, Aceton und Diethylether.
Capping :
Der mit Nucleosid beladene Träger wird mit 10 mg DMAP, 0.5 ml Acetanhydrid und 10 ml Pyridin 0.5 Stunden geschüttelt. Anschließend wird abgesaugt und nacheinander mit DMF, Methanol, Aceton und Diethylether gewaschen.
Im Falle von 5'-O-Dimethoxytrityl-O⁶-[2-(4-nitrophenyl)ethyl]-N₂-phthaloyl-3'-Osuccinyl-2'-desoxyguanosin wird mit einem Gemisch aus 0.5 ml N-Methylimidazol, 0.5 ml Acetanhydrid und 5 ml Pyridin gecappt.

Man erhält folgende Beladungen :

| Träger | Succinat | Beladung [µmol/g] |
|---|---|---|
| 1000 Å | dA^{pth} | 8.9 |
| 1400 Å | dA^{pth} | 12.5 |
| 1400 Å | dGP^{pth/npe} | 10.9 |

### 8.3 Derivatisierung von LCMAA-CPG mit Bernsteinsäureanhydrid :

500 mg LCMAA-CPG 1400 Å werden in 4 ml trockenem Pyridin mit 12 mg DMAP (0.1 mmol) und 400 mg Bernsteinsäureanhydrid 24 Stunden geschüttelt. Man saugt ab und wäscht mit Pyridin und Dichlormethan.

Beladung des Succinyl-LCMAA-CPG mt Phthaloyl-geschützten 5'-O-DMTr-Nucleosiden:

Zu 150 mg succinyliertem LCMAA-CPG, 1.8 mg DMAP (0.015 mmol), 29 mg 1-(3-Dimethylaminopropyl)ethylcabodiimid (0.15 mmol) und 0.015 mmol 5'-O-DMTr-geschützter Phthaloyl-Verbindung werden 3 ml trockenes Pyridin und 12 µl Triethylamin gegeben. Man schüttelt 24 Stunden und gibt anschließend 20 mg Pentachlorphenol (0.07 mmol) zu und schüttelt für weitere 23 Stunden. Dann werden 0.75 ml Piperidin zugegeben, sofort nach 5 Minuten abgesaugt und nacheinander mit Dichlormethan und Diethylether gewaschen.
Capping : Der mit Nucleosid beladene Träger wird mit einem Gemisch aus 0.5 ml N-Methylimidazol, 0.5 ml Acetanhydrid und 5 ml Pyridin 1.5 Stunden geschüttelt und anschließend mit DMF, Methanol, Aceton und Diethylether gewaschen.

Man erhält folgende Beladungen :

| Träger | 5'-O-DMTr-Nucleosid | Beladung [µmol/g] |
|---|---|---|
| 1400 Å | dC^{pth} | 2.2 |
| 1400 Å | dG^{pth/npe} | 3 |

### Beispiel 9:

### Oligonucleotid-Synthese

Unmodifizierte Oligonucleotide wurden auf einem automatischen DNA Synthesizer (Applied Biosystems Model 392) unter Anwendung des Standard Phosphoramidit Synthese-Zyklus synthetisiert. Die Phthaloyl-Strategie erlaubt sowohl die Verwendung des wasserhaltigen Oxidationsgemisches Jod/Pyridin/THF/Wasser als auch wasserfreie Oxidation mit tert-Butyl-Hydroperoxid in Acetonitril. Die Oxidationszeiten betragen für Jod 15 Sekunden bzw. 90 Sekunden für tert-Butyl-Hydroperoxid. Nach Ende der Synthese-Zyklen erfolgt die Schutzgruppenabspaltung noch während sich das Oligonucloetid am Träger befindet mit DBU in N-Methyimidazol bzw. Acetonitril (s.u.) (DBU: 1,8-Diazobicylclo-[6.4.0]-undec7-en) während 15 Minuten bis 12h. Das nun von allen Schutzgruppen freie Oligonucleotid wird nun durch Behandlung mit konzentriertem wässrigen Ammoniak (25 %) vom Träger abgelöst. Das Oligonucleotid liegt nun hochrein in ammoniakalischer Lösung vor und wird durch Lyophilisieren der ammoniakalischen Lösung erhalten. Eine zusätzliche Reinigung durch HPLC bzw. PAGE zur Entfernung der Schutzgruppen ist bei der Pthaloyl-Strategie im Gegensatz zur Acyl-Strategie nicht notwendig.

Die folgenden Sequenzen wurden unter Einsatz der in Beispiel 1-5 beschriebenen Phosphoramidite, und der in Beispiel 8 beschriebenen derivatisierten festen Trägern synthetisiert. Bei den einzelnen Sequenzen wurden die beschriebenen Bedingungen zur Abspaltung der Schutzgruppen erfolgreich variiert.

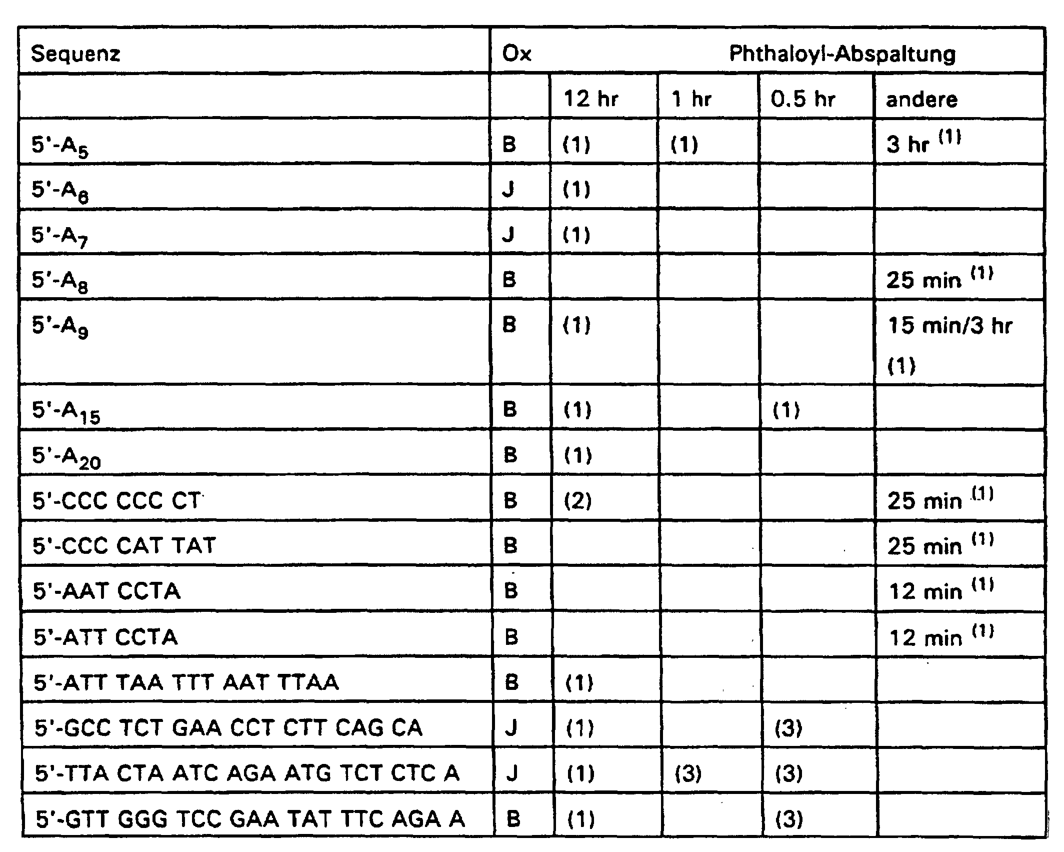

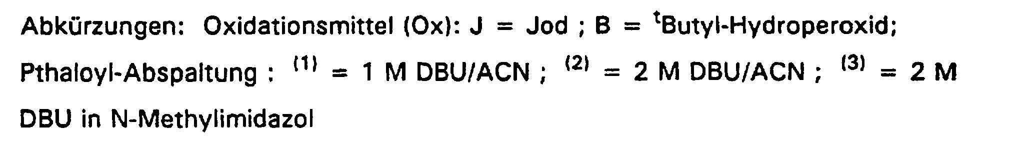

Die folgenden Sequenzen wurden unter Einsatz der in Beispiel 1-5 beschriebenen Phosphoramidite und den in Beispiel 8 beschriebenen festen Trägern synthetisiert. Dabei wurde anstelle von Tetrazol als Aktivator eine Lösung von 0.5M Pyridin-Hydrochlorid in Acetonitril eingesetzt mit Kondensationszeiten von 12 bis 30 Sekunden. Bei den einzelnen Sequenzen wurden die beschriebenen Bedingungen zur Abspaltung der Schutzgruppen erfolgreich variiert.

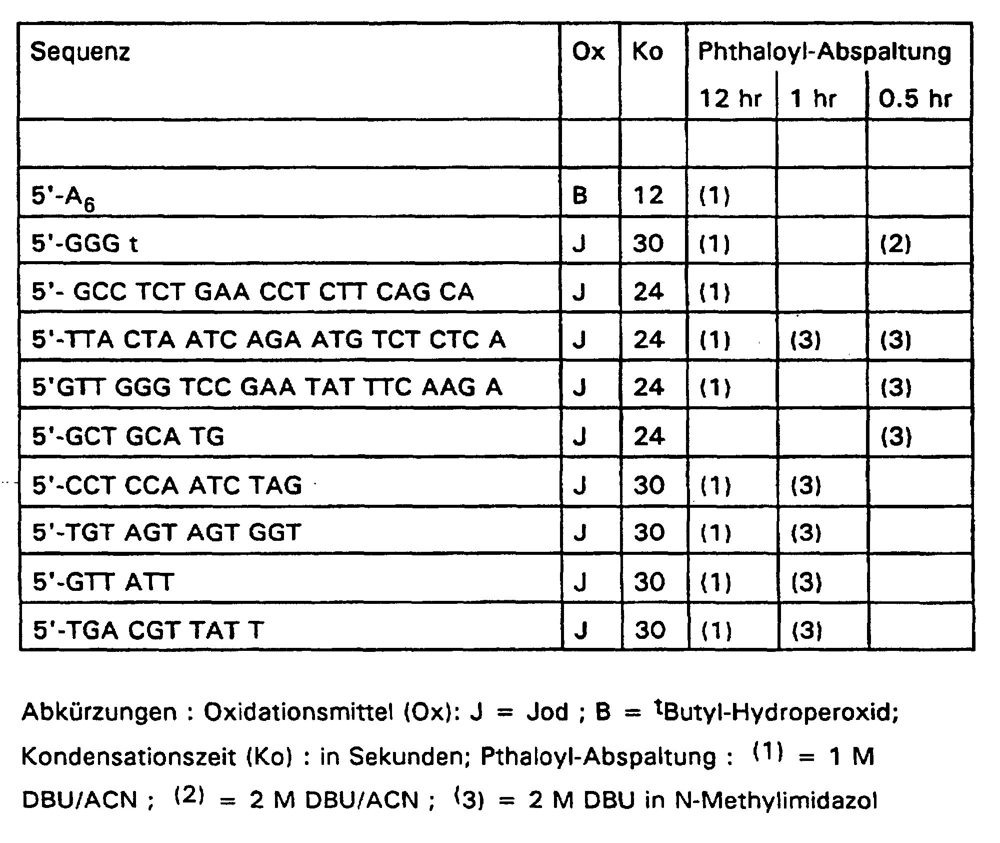

Die hohe Reinheit der Oligonucleotide (>95%) wurde durch HPLC bestätigt.
HPLC : Gradient:
(reversed phase ; RP 18) ; Fluß 1 ml/min
Lösung A : 0.1 N TEAAc pH 7
Lösung B : 0.1 N TEAAc : AcCN 1:1

| Schritt | min | Vol.-% | Vol.-% |
|---|---|---|---|
| 1 | 0 | 95 | 5 |
| 2 | 2 | 95 | 5 |
| 3 | 32 | 60 | 40 |
| 4 | 45 | 0 | 100 |
| 5 | 50 | 95 | 5 |
| 6 | 55 | 95 | 5 |

## Patentansprüche

1. Verfahren zur Herstellung von Oligonukleotiden mittels Festphasensynthese durch
a) sequentiellen Aufbau der Nucleotide an einem festen Träger nach bekannten Methoden, wobei vorhandene exocyclische Aminogruppen an den Nucleobasen durch eine cyclische Diacylgruppe geschützt sind und gegebenenfalls vorhandene Phosphatschutzgruppen durch starke, nicht nukleophile Basen abspaltbar sind,
b) Entschützung der am festen Träger gebundenen Oligonukleotide, und
c) Abspaltung der entschützten Oligonukleotide vom festen Träger,
**dadurch gekennzeichnet, daß** die am festen Träger gebundenen Oligonukleotide in Gegenwart einer starken, nicht nukleophilen Base in einem geeigneten organischen Lösungsmittel entschützt werden.

2. Verfahren gemäß Anspruch 1, **dadurch gekennzeichnet, daß** die Entschützung der am festen Träger gebundenen Oligonukleotiden mit einer 0,1 bis 5 M Lösung von DBU bei 0 bis 70°C für 0,1 bis 16 h erfolgt.

3. Verfahren gemäß Anspruch 1 oder 2, **dadurch gekennzeichnet, daß** die Entschützung der am festen Träger gebundenen Oligonukleotide mit einer 0,3 bis 3 M Lösung von DBU bei 10 bis 40°C für 0,1 bis 2 h erfolgt.

4. Verfahren gemäß einem der Ansprüche 1 bis 3, **dadurch gekennzeichnet, daß** die Entschützung der am festen Träger gebundenen Oligonukleotide mit einer 0,5 bis 2,5 M Lösung von DBU bei 20 bis 30°C für 0,2 bis 1,5 h erfolgt.

5. Verfahren gemäß einem der Ansprüche 1 bis 4 zur Herstellung einer Verbindung der Formel I 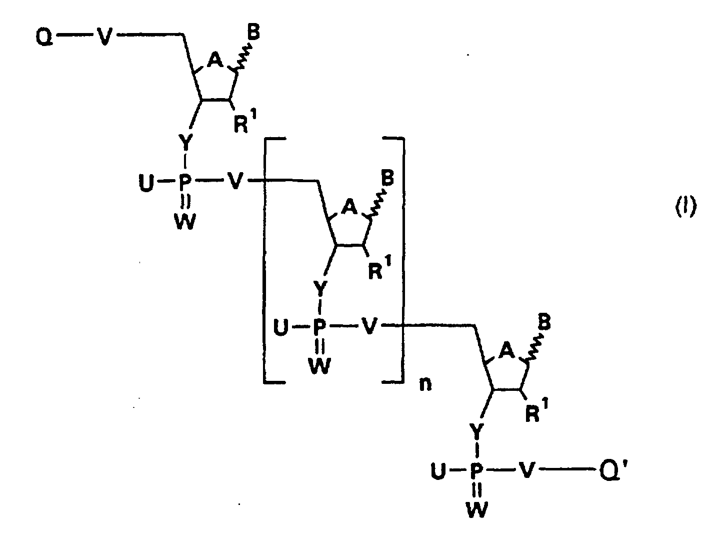 worin
R¹ unabhängig voneinander Wasserstoff, Hydroxy, C₁-C₁₈-Alkoxy, gegebenenfalls ein bis dreifach substituiert mit Hydroxy, oder C₁-C₄-Alkoxy, C₁-C₄-Alkyl-O-(CH₂CH₂O)ₛ worin s eine Zahl von 1 bis 3 bedeutet; O-Allyl, Halogen, Azido oder Amino bedeutet;
A unabhängig voneinander für Oxy, Thioxy oder Methylen steht;
W unabhängig voneinander Oxo, Thioxo oder Selenoxo bedeutet;
V unabhängig voneinander Oxy, Sulfandiyl oder Imino bedeutet;
Y unabhängig voneinander Oxy, Sulfandiyl, Imino oder Methylen bedeutet;
B für eine in der Nucleotidchemie übliche Base steht, wobei mindestens ein B für eine Base steht, die eine exocyclische Aminogruppe aufweist;
n eine ganze Zahl von 1 bis 100 bedeutet;
U unabhängig voneinander Hydroxy, Mercapto, BH₃, SeH, C₁-C₁₈-Alkoxy, vorzugsweise C₁-C₆-Alkoxy, C₁-C₁₈-Alkyl,vorzugsweise C₁-C₆-Alkyl,C₆-C₂₀-Aryl, (C₆-C₁₄)-Aryl-(C₁-C₈)-alkyl, NHR³, NR³R⁴ oder einen Rest der Formel II
(OCH₂CH₂)ₚO(CH₂)qCH₂R² (II)
bedeutet, worin
R³ C₁-C₁₈-Alkyl, vorzugsweise C₁-C₈-Alkyl, C₆-C₂₀-Aryl, (C₆-C₁₄)-Aryl-(C₁-C₈)-alkyl, -(CH₂)_{c}-[NH(CH₂)_{c}]_{d}-NR⁵R⁵, worin c eine ganze Zahl von 2 bis 6 und d eine ganze Zahl von 0 bis 6 ist;
R⁵ unabhängig voneinander Wasserstoff oder C₁-C₆-Alkyl oder C₁-C₄-Alkoxy-C₁-C₆-alkyl, bevorzugt Methoxyethyl, ist;
R⁴ C₁-C₁₈-Alkyl, vorzugsweise C₁-C₈-Alkyl und besonders bevorzugt C₁-C₄-Alkyl, C₆-C₂₀-Aryl oder (C₆-C₁₀)-Aryl-(C₁-C₈)-alkyl bedeutet, oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann;
p eine ganze Zahl von 1 bis 100, bevorzugt 3 bis 10 ist,
q eine ganze Zahl von 0 bis 22, bevorzugt 0 bis 15 ist,
R² Wasserstoff oder eine funktionelle Gruppe wie Hydroxy, Amino, NHR⁶, COOH, CONH₂, COOR⁷ oder Halogen bedeutet, worin
R⁶ C₁-C₄-Alkyl und
R⁷ C₁-C₄-Alkyl, vorzugsweise Methyl bedeutet;
Q und Q' unabhängig voneinander Wasserstoff bedeuten oder für Konjugate stehen, welche die Eigenschaften von Antisense-Oligonucleotiden oder von Tripelhelix bildenden Oligonucleotiden günstig beeinflußen oder als Markierung einer DNA Sonde dienen oder bei der Hybridisierung des Oligonucleotidanalogons an die Target-Nucleinsäure diese unter Bindung oder Quervernetzung angreift;
wobei
sich R¹ und der benachbarte Phosphorylrest sowohl in 2'- und 3'-Stellung oder auch umgekehrt in 3'- und 2'-Stellung befinden können;
jedes Nucleotid in seiner D- bzw. L-Konfiguration vorliegen kann;
die Base B in alpha- bzw. beta-Stellung befinden kann, wobei das Oligonukleotid auch 3'-3'- bzw. 5'-5'-Inversionen beinhalten kann,
**dadurch gekennzeichnet, daß** man
(a) eine Verbindung der Formel III 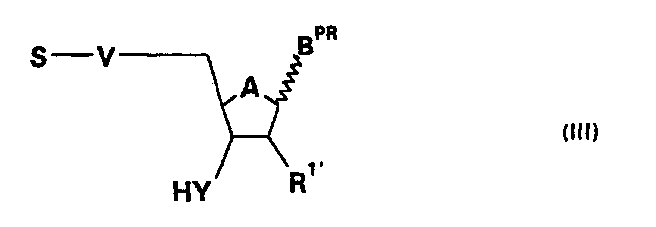
in der A, Y, V, wie oben definiert sind und
R^{1'} wie R¹ definiert ist wobei, wenn R¹ für Hydroxy oder Amino steht R^{1'} eine entsprechend geschützte Gruppe bedeutet,
S für eine im sauren abspaltbare 5'- Schutzgruppe steht;
B^{PR} für eine natürliche oder unnatürliche Nucleobase steht, wobei gegebenenfalls vorhandene exocyclische Aminogruppen durch eine cyclische Diacylgruppe geschützt sind;
nach bekannten Verfahren mit einer Verbindung der Formel IV 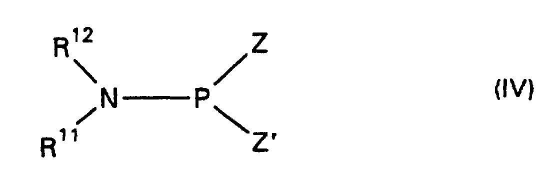 worin
Z' für OR¹³ oder C₁-C₁₈-Alkyl, C₁-C₁₈-Alkoxy, C₆-C₂₀-Aryl, C₆-C₁₄-Aryl-C₁-C₈-Alkyl, bevorzugt für OR¹³, C₁-C₆-Alkyl, C₁-C₆-Alkoxy, C₆-C₂₀-Aryl, C₆-C₁₄-Aryl-C₁-C₈-Alkyl, besonders bevorzugt für OR¹³ steht;
R¹¹ und R¹² gleich oder verschieden sind und C₁-C₈-Alkyl, vorzugsweise Isopropyl, oder C₅-C₁₂-Cycloalkyl, vorzugsweise bis C₈, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁ C₄-Alkyl-Ester, bedeuten; und
R¹³ für eine, durch starke, nicht nukleophile Basen abspaltbare Schutzgruppe steht,
Z für Chlor oder Brom oder einen Rest der Formel NR¹¹R¹², steht wobei R¹¹ und R¹² wie oben definiert sind;
in Gegenwart einer Base, vorzugsweise Pyridin oder einer Mischung von Tetrahydrofuran (THF), Dioxan, Dichlormethan (DCM), Chloroform, und/oder Acetonitril mit einem C₁-C₄-Trialkylamin, vorzugsweise Trimethyl-, Triethyloder Diisopropylethyl-Amin oder, wenn Z ein Rest der Formel NR¹¹R¹² ist, dann in Gegenwart
einer Verbindung der Formel [HNR¹⁴R¹⁵R¹⁶](+) _{A(-)},
wobei R¹⁴, R¹⁵, R¹⁶ gleich oder verschieden voneinander sind und eine C₁-C₄-Alkylgruppe und A Fluor, Chlor, Brom, insbesondere Chlor, bedeuten;
oder Tetrazol oder 5-(C₁-C₄-Alkylthio)-1H-tetrazol, vorzugsweise in Gegenwart von Tetrazol oder Pyridinhydrochlorid, zu einer Verbindung der Formel V umsetzt 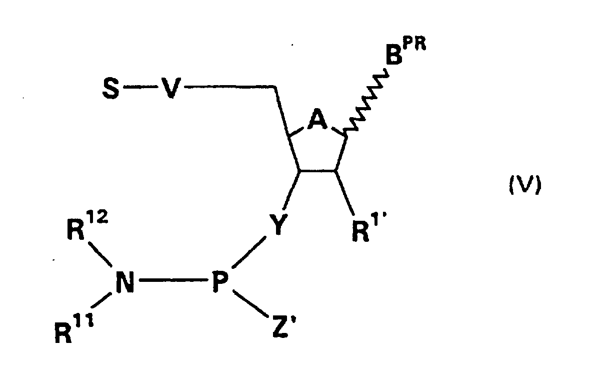 worin S, V, Y,A, B^{PR}, Z', R^{1'}, R¹¹, R¹² wie oben-definiert sind;
(b) Verbindungen der Formel III nach bekannten Verfahren mit 1 bis 10 Equivalenten, bevorzugt mit 1 bis 2 Equivalenten eines Linkers in einem geeigneten organischen Lösungsmittel, gegebenenfalls nach Zugabe eines Katalysators zu einer Verbindung der Formel VI 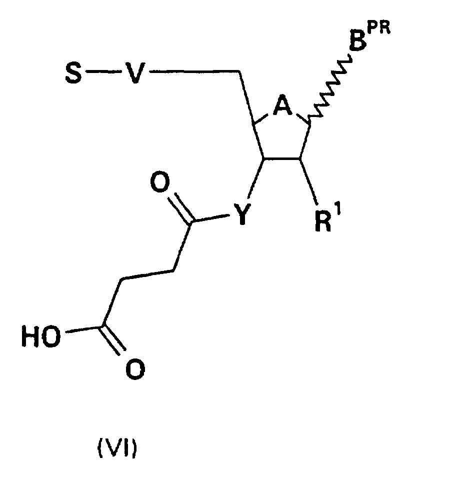 wobei S, V, Y,A, B^{PR} und R¹ wie oben definiert sind, umsetzt und anschließend nach bekannten Verfahren aufgearbeitet werden, wobei der Bernsteinsäure-Rest in 3'-Position als Linker zu dem in der Synthese eingesetzten Polymerträger dient.
(c) die Verbindung der Formel VI nach bekannten Verfahren auf einen festen Träger SS in einem geeigneten Lösungsmittel koppelt, wobei Verbindungen der Formel VII erhalten werden; 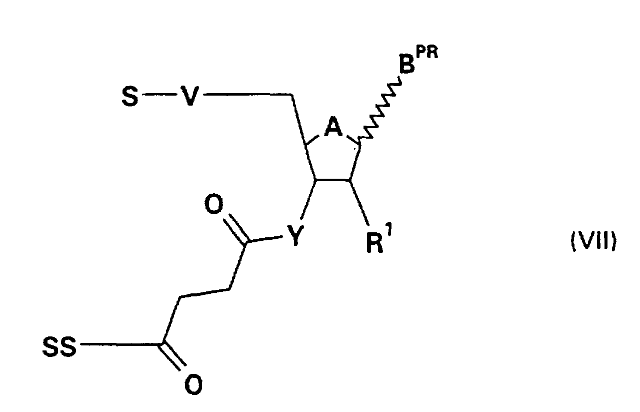
(d) die 5'-Schutzgruppe von VII nach bekannten Verfahren abspaltet;
(e) die erhaltene Verbindung mit einer Verbindung der Formel V in einem geeigneten organischen Lösungsmittel, bevorzugt Acetonitril, in Gegenwart einer Verbindung der Formel [HNR¹⁴R¹⁵R¹⁶]⁽⁺⁾ A⁽⁻⁾ ,die wie oben definiert ist, oder Tetrazol, 5-(C₁-C₄-Alkylthio)-1H-tetrazol oder 5-(C₆-C₁₂-Aryl)-1H-tetrazol, vorzugsweise in Gegenwart von Tetrazol oder Pyridin-Hydrochlorid, umsetzt und
(f) die erhaltene Verbindung nach bekannten Verfahren oxidiert, gegebenenfalls auch in Gegenwart weiterer organischer Lösungsmittel,
(g) die Reaktionsschritte d - f bis zur gewünschten Kettenlänge wiederholt;
(h) die so erhaltene Verbindung durch Behandlung mit DBU in einem geeigneten organischen Lösungsmittel entschützt; und
(i) das Oligonucleotid nach bekannten Verfahren vom Träger abspaltet.

6. Verfahren gemäß Anspruch 5, **dadurch gekennzeichnet daß** nicht abreagierte Verbindungen aus Schritt (d) durch einen capping-Schritt deaktiviert werden.

7. Verfahren gemäß einem der Ansprüche 5 oder 6, wobei in Verbindungen der Formeln (III), (V), (VI) und (VII) B^{PR} unabbhängig voneinander 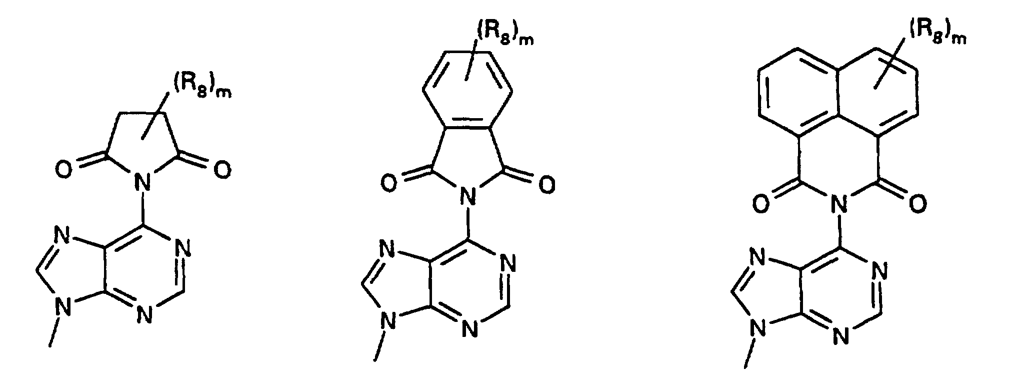 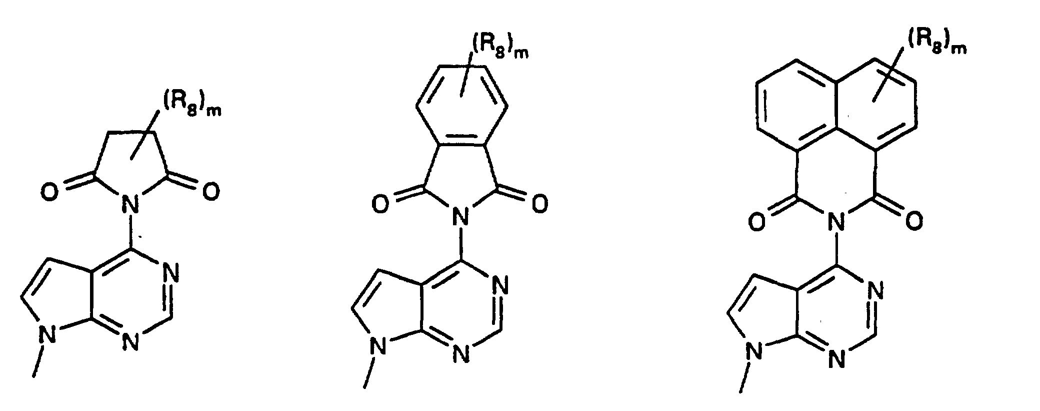 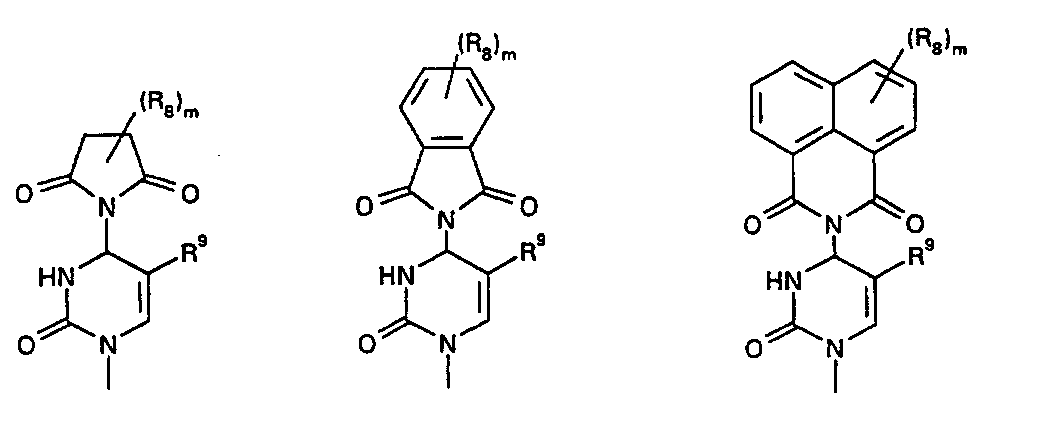 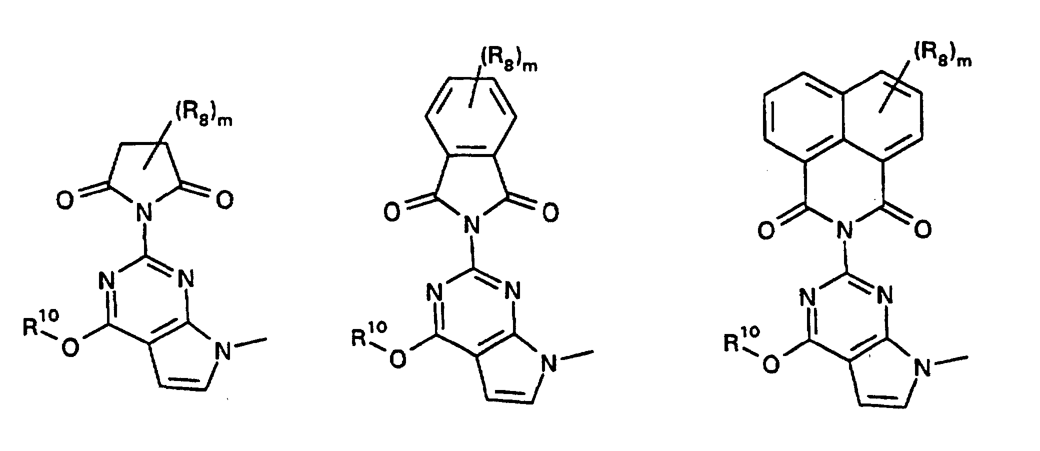 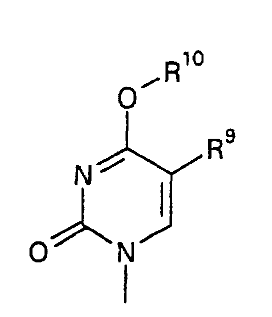 bedeutet,
worin
m für eine Zahl von Null bis vier, bevorzugt für Null steht, die Reste
R⁸ unabhängig voneinander für Wasserstoff, Fluor, Chlor, Brom, Nitro, C₁-C₄-Alkyl, C₁-C₄-Alkoxy, CN steht,
R⁹ unabhängig voneinander Wasserstoff, C₁-C₆-Alkyl, C₂-C₆-(1-Alkin), bevorzugt 1-Propinyl und 1-Hexinyl oder Fluor bedeutet, und
R¹⁰ unbahängig voneinander für Wasserstoff oder einer β-eliminierbare Schutzgruppen stehen.

8. Verfahren gemäß einem der Ansprüche 5 bis 7 zur Herstellung einer Verbindung der Formel I worin
R¹ Wasserstoff, Hydroxy, C₁-C₄-Alkoxy oder Fluor bedeutet;
A für Oxy steht;
W Oxo oder Thioxo bedeutet;
V Oxy bedeutet;
Y Oxy bedeutet;
B unabhängig voneinander für Adenin, Cytosin, Guanin, Uracil, Thymin, 5-Propinuracil, 5-Propincytosin, 5-Hexinuracil oder 5-Hexincytosin steht
n eine ganze Zahl von 5 bis 40 bedeutet;
U für für Hydroxy, Mercapto, C₁-C₆-Alkoxy, C₁-C₆-Alkyl, NR³R⁴ oder NHR³ steht, worin
R³ für C₁-C₈-Alkyl oder Methoxyethyl steht
R₄ C₁-C₈-Alkyl, C₆-C₂₀-Aryl oder (C₆-C₁₀)-Aryl-(C₁-C₈)-alkyl bedeutet oder im Falle von NR³R⁴ zusammen mit R³ und dem sie tragenden Stickstoffatom einen 5-6-gliedrigen heterozyklischen Ring bedeutet, der zusätzlich ein weiteres Heteroatom aus der Reihe O, S, N enthalten kann,
Q und Q' unabhängig voneinander Wasserstoff bedeuten.

9. Verfahren gemäß Anspruch 8, wobei
R¹ Wasserstoff bedeutet
n eine ganze Zahl von 5 bis 30 bedeutet
u Hydroxy oder C₁-C₆-Alkyl
Q und Q' Wasserstoff bedeuten
und die restlichen Variablen wie in Anspruch 7 definiert sind.

10. Verbindung der Formel V 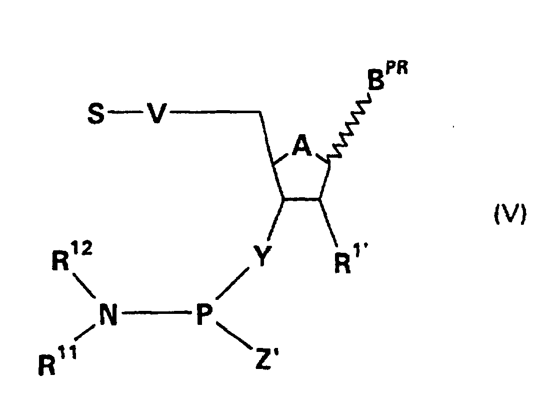 worin
R^{1'} unabhängig voneinander Wasserstoff, C₁-C₁₈-Alkoxy, gegebenenfalls ein bis dreifach substituiert mit Hydroxy, oder C₁-C₄-Alkoxy, C₁-C₄-Alkyl-O-(CH₂CH₂O)₁₋₃, O-Allyl, Fluor, Chlor, Azido oder eine geschützte Hydroxyoder Aminogruppe bedeutet;
A für Oxy, Thioxy oder Methylen steht;
V Oxy, Sulfandiyl oder Imino bedeutet;
Y Oxy, Sulfandiyl, Imino oder Methylen bedeutet;
S für eine im sauren abspaltbare 5'-Schutzgruppe steht;
B^{PR} für eine natürliche oder unnatürliche Nucleobase mit exocyclischer Aminogruppe steht, wobei die exocyclischen Aminogruppen durch eine cyclische Diacylgruppe geschützt sind;
Z' für OR¹³, C₁-C₁₈-Alkyl, C₁-C₁₈-Alkoxy, C₆-C₂₀-Aryl, C₆-C₁₄-Aryl-C₁-C₈-Alkyl, steht;
R¹¹ und R¹² gleich oder verschieden sind und C₁-C₈-Alkyl, vorzugsweise Isopropyl, oder C₅-C₁₂-Cycloalkyl, vorzugsweise bis C8, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-Alkyl-Ester, bedeuten; und
R¹³ für para-Nitrophenylethyl oder 2-Cyanoethyl steht.

11. Verbindung der Formel V gemäß Anspruch 10, worin B^{PR} 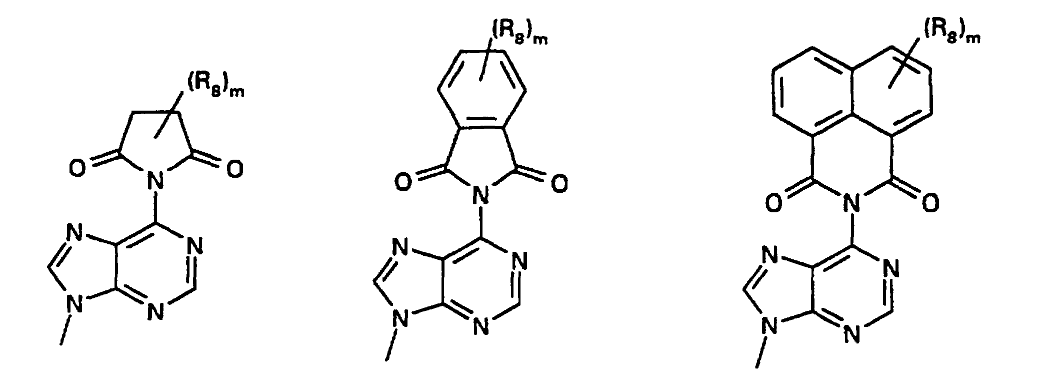 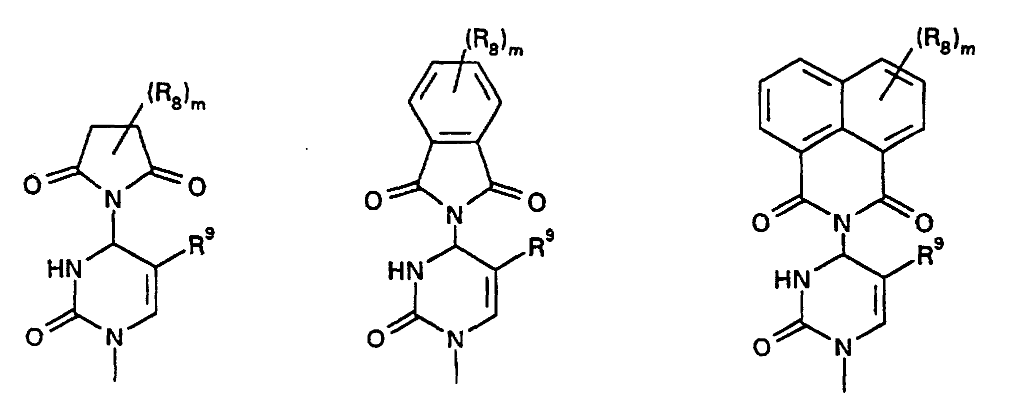 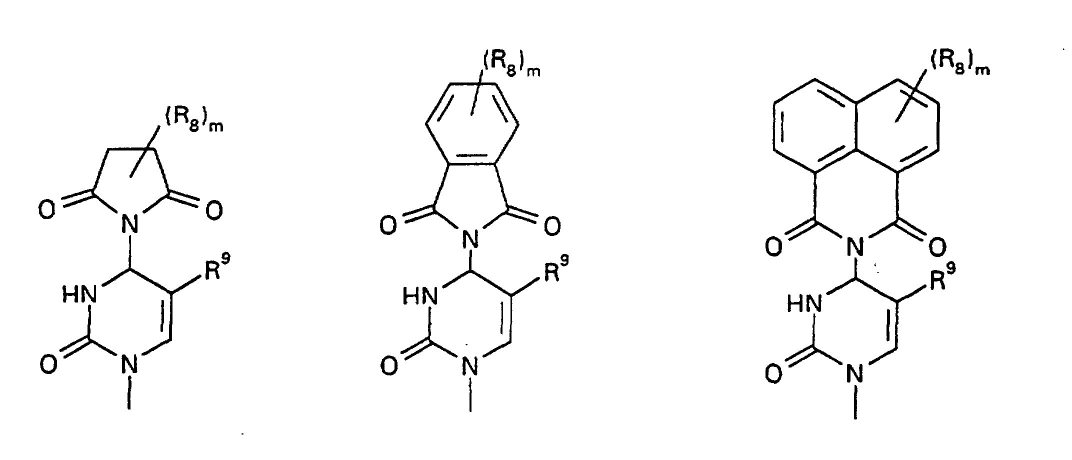 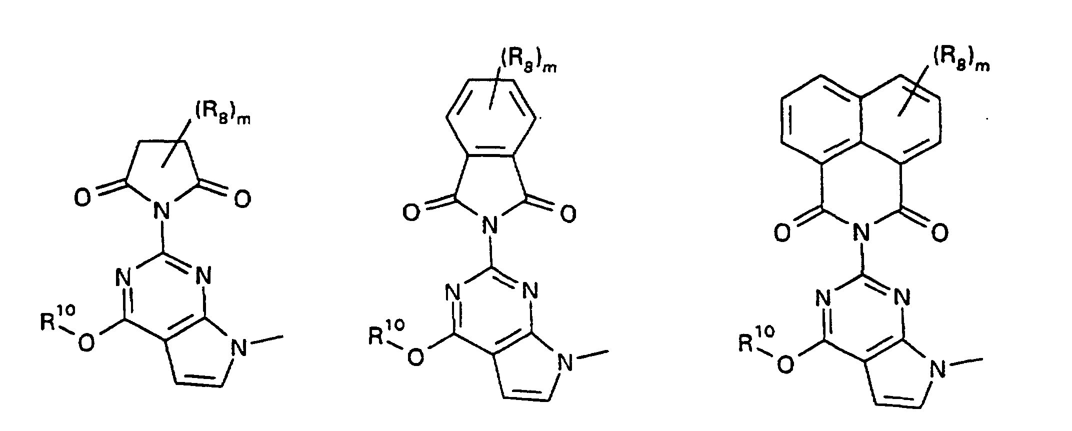 bedeutet,
worin
m für eine Zahl von Null bis vier, bevorzugt für Null steht; und
R⁸ unabhängig voneinander für Wasserstoff, Fluor, Chlor, Brom, Nitro, C₁-C₄-Alkyl, C₁-C₄-Alkoxy, CN steht,
und R⁹ und R¹⁰ wie in Anspruch 7 definiert sind.

12. Verbindung der Formel V gemäß Anspruch 10 oder 11, worin
R^{1'} Wasserstoff, eine geschützte Hydroxy-Gruppe, C₁-C₄-Alkoxy oder Fluor bedeutet,
A für Oxy steht;
V Oxy bedeutet;
Y Oxy bedeutet;
Z' OR¹³, C₁-C₆-Alkyl, C₁-C₆-Alkoxy, C₆-C₂₀-Aryl, C₆-C₁₄-Aryl-C₁-C₈-Alkyl, bevorzugt OR¹³ bedeutet.

## Revendications

1. Procédé pour la préparation d'oligonucléotides par la synthèse en phase solide par
a) assemblage séquentielle des nucléotides sur un substrat solide selon des méthodes connues, les groupes amino exocycliques présents sur les bases nucléiques étant protégés par un groupe diacyle cyclique et les groupe protecteurs phosphate éventuellement présents sont éliminables par des bases fortes, non nucléophiles,
b) déprotection des oligonucléotides liés au substrat solide, et
c) élimination des oligonucléotides déprotégés du substrat solide,
**caractérisé en ce que** les oligonucléotides liés au substrat solide sont déprotégés en présence d'une base forte, non nucléophile, dans un solvant organique approprié.

2. Procédé selon la revendication 1, **caractérisé en ce qu'**on réalise la déprotection des oligonucléotides liés au substrat solide à l'aide d'une une solution de DBU de 0,1 à 5M à une température de 0 à 70°C pendant 0,1 à 16 heures.

3. Procédé selon la revendication 1 ou 2, **caractérisé en ce qu'**on met en oeuvre la déprotection de l'oligonucléotide lié au substrat solide à l'aide d'une solution de DBU de 0,3 à 3 M, à une température de 10 à 40°C pendant 0,1 à 2 heures.

4. Procédé selon l'une des revendications 1 à 3, **caractérisé en ce qu'**on met en oeuvre la déprotection des oligonucléotides liés au substrat solide à l'aide d'une solution de DBU de 0,5 à 2,5 M, à une température de 20 à 30°C pendant 0,2 à 1,5 heure.

5. Procédé selon l'une des revendications 1 à 4 pour la préparation d'un composé de formule I 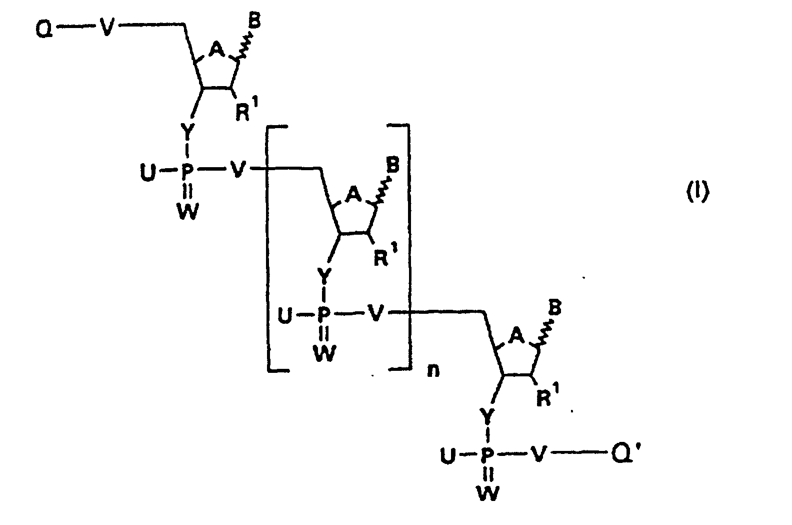 où
R¹ représentent, indépendamment les uns des autres, un atome d'hydrogène, des groupes hydroxy, alkoxy en C₁-C₁₈, éventuellement substitué par un substituant hydroxy, ou alkoxy en C₁-C₄, (alkyl en C₁-C₄)-O-(CH₂-CH₂-O)ₛ, où s est un nombre de 1 à 3 ; O-allyle, halogène, azido ou amino ;
A représentent, indépendamment les uns des autres, des groupes oxy, thioxy ou méthylène ;
W représentent, indépendamment les uns des autres, des groupes oxo, thioxo ou sélénoxo ;
V représentent, indépendamment les uns des autres, des groupes oxy, sulfanediyle ou imino ;
Y représentent, indépendamment les uns des autres, des groupes oxy, sulfanediyle, imino ou méthylène ;
B représente une base usuelle en chimie des nucléotides, au moins un B représentant une base, qui présente un groupe amino exocyclique ;
n est un entier de 1 à 100 ;
U représentent, indépendamment les uns des autres, des groupes hydroxy, mercapto, BH₃, SeH, alkoxy en C₁-C₁₈, de préférence alkoxy en C₁-C₆, alkyle en C₁-C₁₈, de préférence alkyle en C₁-C₆, aryle en C₆-C₂₀, (aryl en C₆-C₁₄)-alkyle en C₁-C₈, NHR³, NR³R⁴ ou un reste de formule II
(OCH₂CH₂)ₚO(CH₂)_{q}CH₂R² (II)
où
R³ représente des groupes alkyle en C₁-C₁₈, de préférence alkyle en C₁-C₈, aryle en C₆-C₂₀, (aryl en C₆-C₁₄)-alkyle en C₁-C₈, -(CH₂)_{c}-[NH(CH₂)_{c}]_{d}-NR⁵R⁵, où c est un entier de 2 à 6 et d est un entier de 0 à 6 ;
R⁵ représentent, indépendamment les uns des autres, un atome d'hydrogène ou un groupe alkyle en C₁-C₆ ou (alkoxy en C₁-C₄)-alkyle en C₁-C₆, de préférence méthoxyéthyle ;
R⁴ représente des groupes alkyle en C₁-C₁₈, de préférence alkyle en C₁-C₈ et de manière particulièrement préférée alkyle en C₁-C₄, aryle en C₆-C₂₀ ou (aryl en C₆-C₁₀)-alkyle en C₁-C₈, ou dans le cas de NR³R⁴ conjointement avec R³ et l'atome d'azote qui les porte représente un cycle hétérocyclique de 5 à 6 chaînons, qui peut présenter de plus un autre hétéroatome pris dans le groupe des atomes O, S, N ;
P est un entier de 1 à 100, de préférence de 3 à 10 ;
q est un entier de 0 à 22, de préférence de 0 à 15 ;
R² représente un atome d'hydrogène ou un groupe fonctionnel comme hydroxy, amino, NHR⁶, COOH, CONH₂, COOR⁷ ou halogène, où
R⁶ représente un groupe alkyle en C₁-C₄, et
R⁷ représente un groupe alkyle en C₁-C₄, de préférence méthyle ;
Q et Q' représentent, indépendamment l'un de l'autre, un atome d'hydrogène ou des conjugués, qui influencent favorablement les propriétés des oligonucléotides anti-sens ou des oligonucléotides formant une triple hélice ou comme marqueurs d'une sonde d'ADN ou s'attachent à l'acide nucléique cible dans l'hybridation des analogues d'oligonucléotides sur l'acide nucléique cible en formant une liaison ou une réticulation ;
où
R¹ et le reste phosphoryle adjacent peuvent se trouver en position 2' et 3' ou aussi inversement en position 3' et 2' ;
chaque nucléotide peut se présenter sous sa configuration D ou L ;
la base B peut se trouver en position alpha ou bêta, l'oligonucléotide peut aussi comprendre des inversions 3'-3' ou 5'-5',
**caractérisé en ce qu'**on fait réagir
(a) un composé de formule III 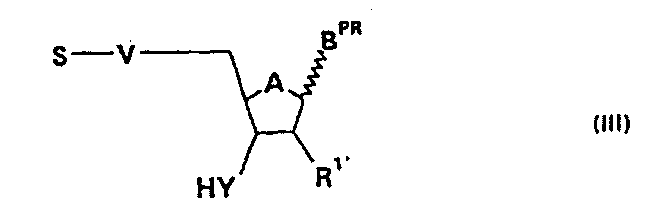 dans laquelle A, Y, V sont définis comme ci-dessus et
R^{1'} comme R¹ sont définis comme ci-dessus, lorsque R¹ représente un groupe ou amino, R^{1'} représente un groupe protégé de façon correspondante,
S représente un groupe protecteur 5' éliminable en milieu acide ;
B^{PR} représente une base nucléique naturelle ou synthétique, des groupes amino exocycliques éventuellement présents peuvent être protégés par un groupe diacyle cyclique ;
selon des procédés connus, sur un composé de formule IV 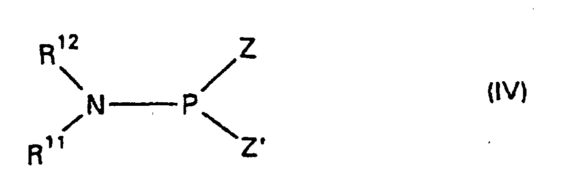 où
Z' représente des groupes OR¹³ ou alkyle en C₁-C₁₈, alkoxy en C₁-C₁₈, aryle en C₆-C₂₀, (aryl en C₆-C₁₄)-alkyle en C₁-C₈, de préférence OR¹³, alkyle en C₁-C₆, alkoxy en C₁-C₆, aryle en C₆-C₂₀, (aryl en C₆-C₁₄)-alkyle en C₁-C₈, de manière particulièrement préférée OR¹³ ;
R¹¹ et R¹² sont identiques ou différents et représentent des groupes alkyle en C₁-C₈, de préférence isopropyle, ou cycloalkyle en C₅-C₁₂, de préférence jusqu'à C₈, benzyle ou phényle ou forment conjointement avec l'atome d'azote auquel ils sont liés un cycle hétérocyclique saturé ou insaturé, éventuellement avec d'autres hétéroatomes comme, par exemple morpholine, et des substituants, tels que des esters OC(O)O-alkyle en C₁-C₄ ; et
R¹³ représente un groupe protecteur éliminable par des bases fortes, non nucléophiles,
Z représente un atome de chlore ou de brome ou un reste de formule NR¹¹R¹², R¹¹ et R¹² étant définis comme ci-dessus ;
en présence d'une base, de préférence la pyridine ou un mélange de tétrahydrofuranne (THF), de dioxanne, de dichlorométhane (DCM), de chloroforme et/ou d'acétonitrile sur une tri-(alkyl en C₁-C₄)amine, de préférence la triméthyl-, la triéthyl- ou la diisopropyléthylamine ou, lorsque Z représente un reste de formule NR¹¹R¹², alors en présence d'un composé de formule [HNR¹⁴R¹⁵R¹⁶]⁽⁺⁾A⁽⁻⁾,
R¹⁴, R¹⁵, R¹⁶ étant identiques ou différents et représentent un groupe alkyle en C₁-C₄ et A représente un atome de fluor, de chlore, de brome, en particulier de chlore ;
ou d'un tétrazole ou 5-(alkyl en C₁-C₄)-thio-1H-tétrazole, de préférence en présence de tétrazole ou de chlorhydrate de pyridine, pour obtenir un composé de formule V 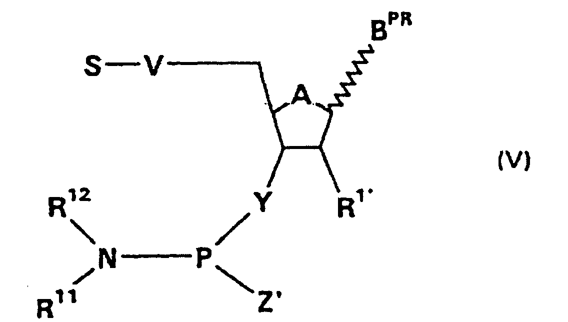 où S, V, Y, A, B^{PR}, Z', R^{1'}, R¹¹, R¹² sont définis comme ci-dessus ;
(b) on fait réagir des composés de formule III selon des procédés connus sur 1 à 10 équivalents, de préférence 1 à 2 équivalents, d'un lieur dans un solvant organique approprié, éventuellement après l'ajout d'un catalyseur, pour obtenir un composé de formule VI 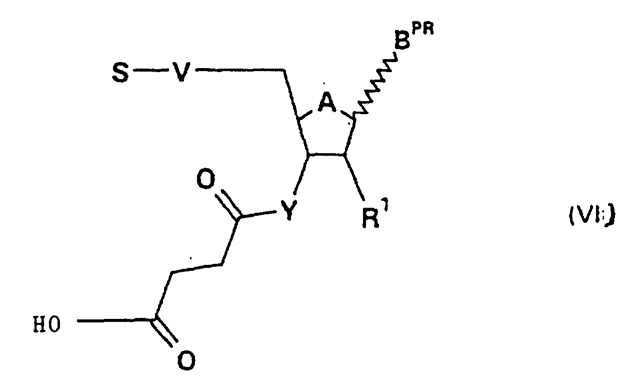 S, V, Y, A, B^{PR} et R¹ sont définis comme ci-dessus, et ensuite on complète le traitement selon des procédés connus, le reste acide succinique en position 3' servant de lieur pour le substrat polymère utilisé dans la synthèse.
(c) on couple le composé de formule VI selon des procédés connus à un substrat solide SS dans un solvant approprié, en obtenant des composés de formule VII ; 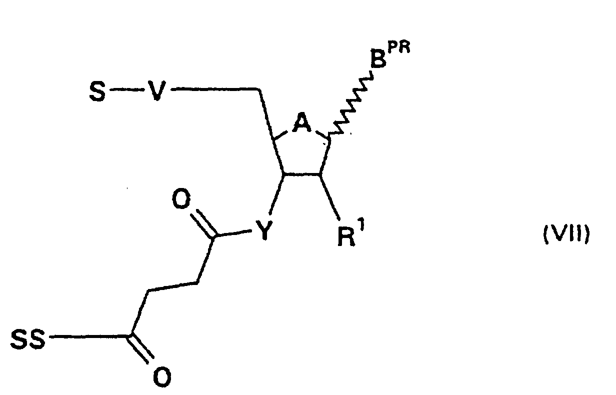
(d) on élimine le groupe protecteur 5' à partir de VII selon des procédés connus ;
(e) on fait réagir le composé obtenu sur un composé de formule V dans un solvant organique approprié, de préférence l'acétonitrile, en présence d'un composé de formule
[HNR¹⁴R¹⁵R¹⁶]⁽⁺⁾A⁽⁻⁾ qui est défini comme ci-dessus, ou de tétrazole, de 5-(alkyl en C₁-C₄)thio-1H-tétrazole ou 5-(aryl en C₆-C₁₂)-1H-tétrazole, de préférence en présence de tétrazole ou de chlorhydrate de pyridine, et
(f) on oxyde le composé obtenu selon des procédés connus, éventuellement également en présence d'autres solvants organiques,
(g) on répète les étapes réactionnelles d-f jusqu'à l'obtention de la longueur de chaîne voulue ;
(h) on déprotège le composé ainsi obtenu par traitement par DBU dans un solvant organique approprié ; et
(i) on sépare l'oligonucléotide selon des procédés connus du substrat.

6. Procédé selon la revendication 5, **caractérisé en ce qu'**on désactive les composés n'ayant pas réagi de l'étape (d) par une étape de "capping".

7. Procédé selon l'une des revendications 5 ou 6, où dans les composés de formules (III), (V), (VI) et (VII), B^{PR} représente, indépendamment l'un de l'autre, 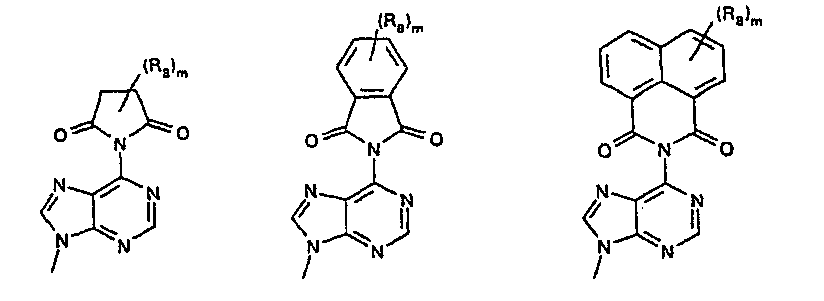 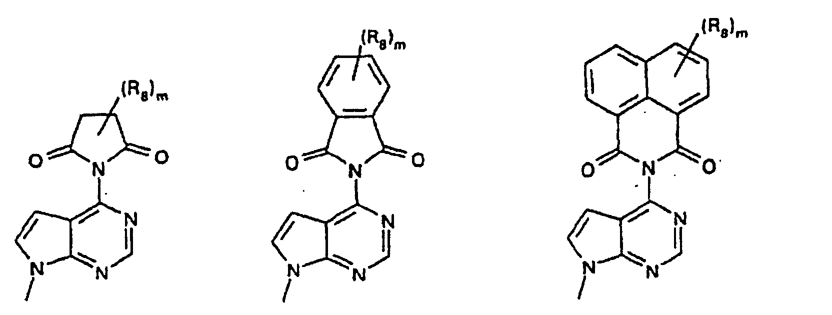 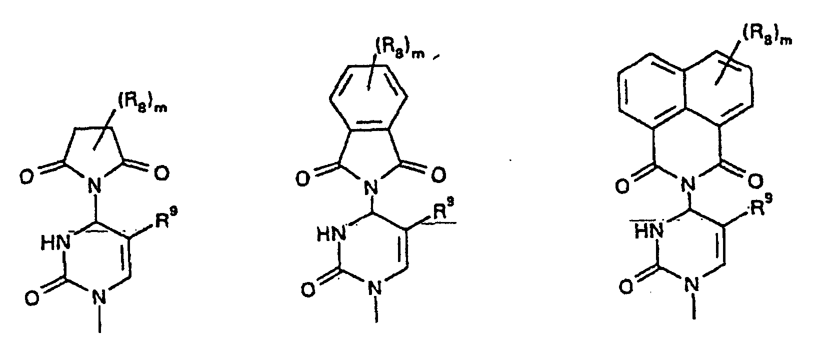 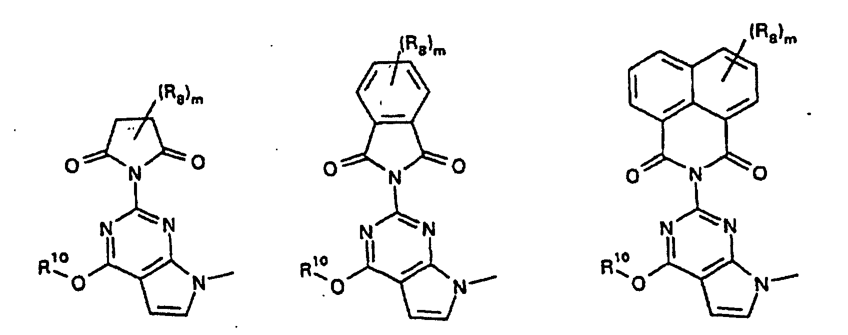 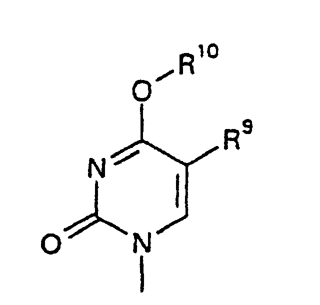 où
m est un nombre de 0 à 4, de préférence valant zéro, les restes
R⁸ représentent, indépendamment les uns des autres, des atomes d'hydrogène, de fluor, de chlore, de brome, des groupes nitro, alkyle en C₁-C₄, alkoxy en C₁-C₄, CN,
R⁹ représentent, indépendamment les uns des autres, un atome d'hydrogène, des groupes alkyle en C₁-C₆, 1-alcyne en C₂-C₆, de préférence 1-propynyle et 1-hexynyle ou un atome de fluor, et
R¹⁰ représentent, indépendamment les uns des autres, un atome d'hydrogène ou un groupe protecteur éliminable par élimination β.

8. Procédé selon l'une des revendications 5 à 7 pour la préparation d'un composé de formule I, où
R¹ représente un atome d'hydrogène, des groupes hydroxy, alkoxy en C₁-C₄ ou un atome de fluor ;
A représente un groupe oxy ;
W représente un groupe oxo ou thioxo ;
V représente un groupe oxy ;
Y représente un groupe oxy ;
B représentent, indépendamment les uns des autres, des adénine, cytosine, guanine, uracile, thymine, 5-propionuracile, 5-propynecytosine, 5-hexynuracile ou 5-hexynecytosine,
n est un entier de 5 à 40 ;
U représente des groupes hydroxy, mercapto, alkoxy en C₁-C₆, alkyle en C₁-C₆, NR³R⁴ ou NHR³, où
R³ représente un groupe alkyle en C₁-C₈ ou méthoxyléthyle,
R⁴ représente des groupes alkyle en C₁-C₈, aryle en C₆-C₂₀ ou (aryl en C₆-C₁₀)-alkyle en C₁-C₈, ou dans le cas de NR³R⁴ conjointement avec R³ et l'atome d'azote qui les porte représente un cycle hétérocyclique de 5 à 6 chaînons, qui peut présenter de plus un autre hétéroatome pris dans le groupe des atomes de O, N, S,
Q et Q' représentent, indépendamment l'un de l'autres, un atome d'hydrogène.

9. Procédé selon la revendication 8, où
R¹ représente un atome d'hydrogène,
n est un entier de 5 à 30,
U représente un groupe hydroxy ou alkyle en C₁-C₆,
Q et Q' représentent un atome d'hydrogène
et les autres variables sont définies comme à la revendication 7.

10. Composé de formule V 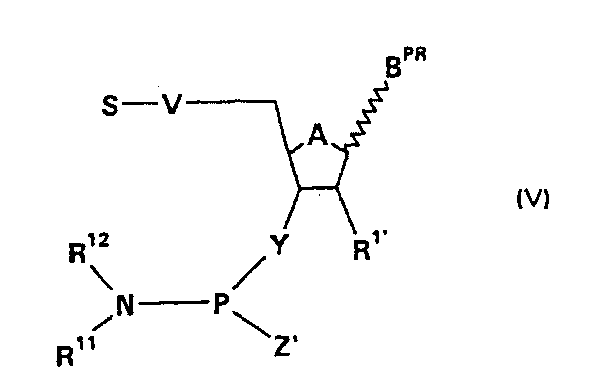 où
R^{1'} représentent, indépendamment les uns des autres, un atome d'hydrogène, des groupes alkoxy en C₁-C₁₈, éventuellement substitué une à trois fois, par un substituant hydroxy, ou alkoxy en C₁-C₄, (alkyl en C₁-C₄)-O-(CH₂CH₂O)₁₋₃, O-allyle, fluor, chlore, azido ou un groupe hydroxy ou amino protégé ;
A représente des groupes oxy, thioxy ou méthylène ;
V représente des groupes oxy, sulfanediyle ou imino ;
Y représente des groupes oxy, sulfanediyle, imino ou méthylène ;
S représente un groupe protecteur 5' éliminable en milieu acide ;
B^{PR} représente une base nucléique naturelle ou synthétique avec un groupe amino exocyclique, les groupes amino exocycliques sont protégés par un groupe diacyle cyclique ;
Z' représente des groupes OR¹³, alkyle en C₁-C₁₈, alkoxy en C₁-C₁₈, aryle en C₆-C₂₀, (aryl en C₆-C₁₄)-alkyle en C₁-C₈ ;
R¹¹ et R¹² sont identiques ou différents et représentent un groupe alkyle en C₁-C₈, de préférence isopropyle, ou cycloalkyle en C₅-C₁₂ de préférence en C₈, benzyle ou phényle ou forment conjointement avec l'atome d'azote auquel ils sont liés un cycle hétérocyclique saturé ou insaturé, éventuellement présentant d'autres hétéroatomes, comme par exemple morpholine, et des substituants tels que des esters OC(O)O-alkyle en C₁-C₄ ; et
R¹³ représente un groupe para-nitrophényléthyle ou 2-cyanoéthyle.

11. Composé de formule V selon la revendication 10, où B^{PR} représente 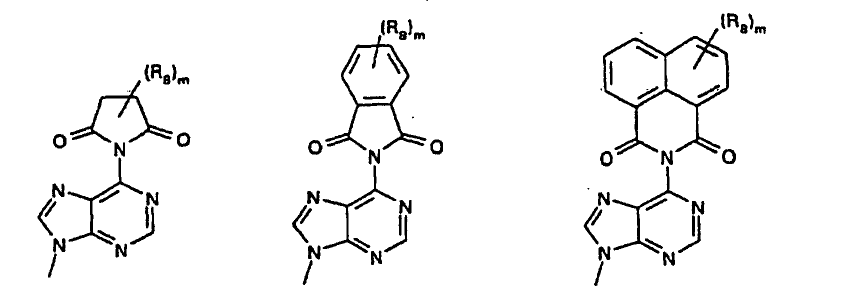 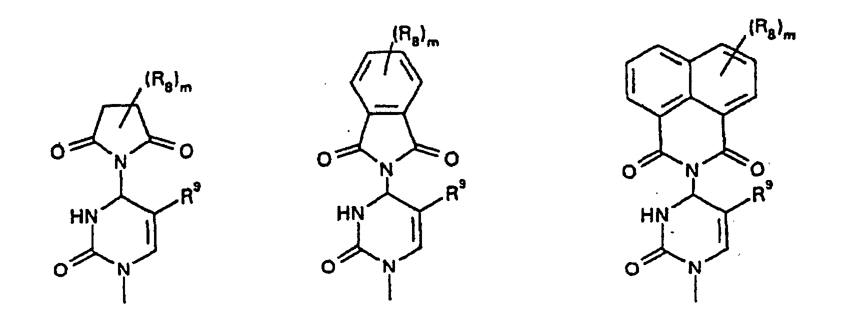 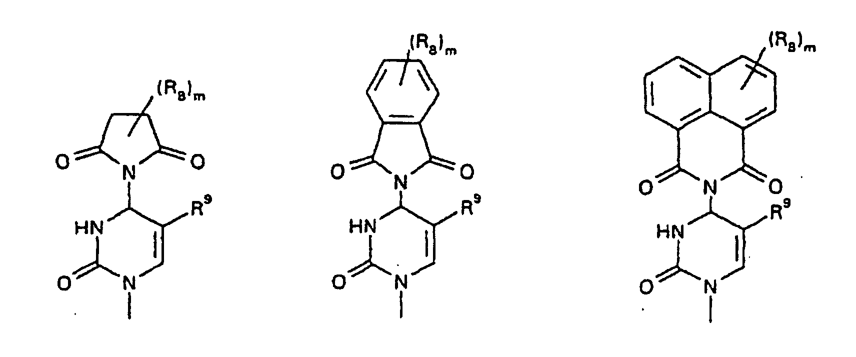 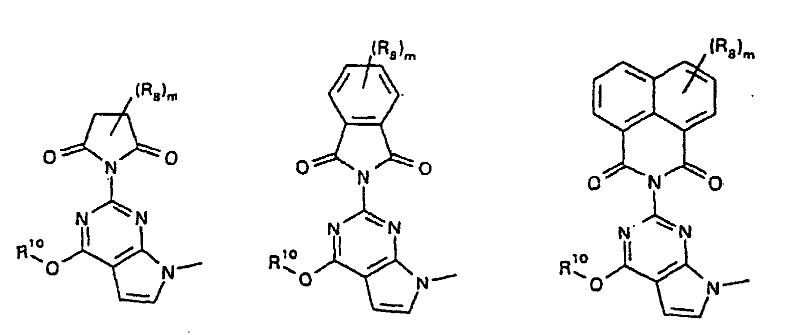 où
m est un nombre de 0 à 4, de préférence zéro ; et
R⁸ représentent, indépendamment les uns des autres, des atomes d'hydrogène, de fluor, de chlore, de brome, des groupes nitro, alkyle en C₁-C₄, alkoxy en C₁-C₄, CN, et
R⁹ et R¹⁰ sont définis comme à la revendication 7.

12. Composé de formule V selon la revendication 10 ou 11, où
R^{1'} représente un atome d'hydrogène, un groupe hydroxy protégé, un groupe alkoxy en C₁-C₄ ou un atome de fluor,
A représente un groupe oxy ;
V représente un groupe oxy ;
T représente un groupe oxy ;
Z' représente des groupes OR¹³, alkyle en C₁-C₆, alkoxy en C₁-C₆, aryle en C₆-C₂₀, (aryl en C₆-C₁₄)-alkyle en C₁-C₈, de préférence OR¹³.

## Claims

1. A process for preparing oligonucleotides by means of solid phase synthesis by
a) sequentially synthesizing the nucleotides on a solid support in accordance with known methods, with exocyclic amino groups which are present on the nucleobases being protected by a cyclic diacyl group and with it being possible to eliminate any phosphate protecting groups which are present with strong, nonnucleophilic bases,
b) deprotecting the oligonucleotides which are bound to the solid support, and
c) cleaving the deprotected oligonucleotides from the solid support,
which comprises deprotecting the oligonucleotides, which are bound to the solid support, in the presence of a strong, nonnucleophilic base in a suitable organic solvent.

2. The process as claimed in claim 1, wherein the oligonucleotides, which are bound to the solid support, are deprotected, at from 0 to 70°C and for from 0.1 to 16 h, with an 0.1 to 5 M solution of DBU.

3. The process as claimed in claim 1 or 2, wherein the oligonucleotides, which are bound to the solid support, are deprotected, at from 10 to 40°C and for from 0.1 to 2 h, with an 0.3 to 3 M solution of DBU.

4. The process as claimed in one of claims 1 to 3, wherein the oligonucleotides, which are bound to the solid support, are deprotected, at from 20 to 30°C and for from 0.2 to 1.5 h, with an 0.5 to 2.5 M solution of DBU.

5. The process as claimed in one of claims 1 to 4 for preparing a compound of the formula I 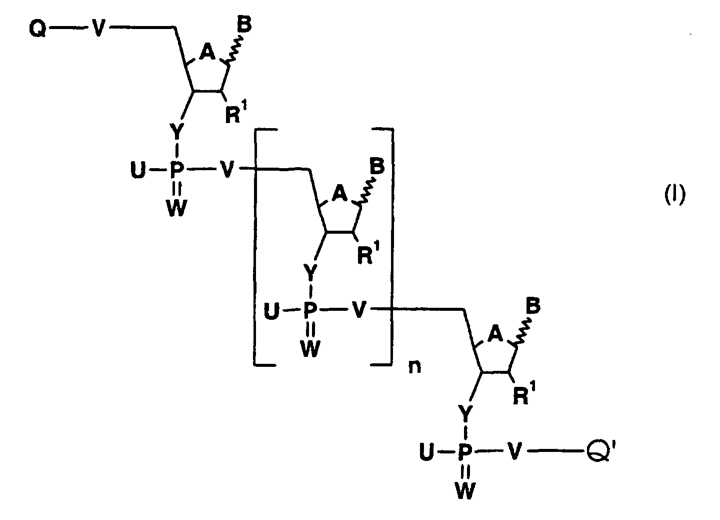 in which
R¹ is, independently of each other, hydrogen, hydroxyl, C₁-C₁₈-alkoxy, which is optionally substituted one to three times by hydroxyl, or C₁-C₄-alkoxy, C₁-C₄-alkyl-O- (CH₂CH₂O)ₛ, in which s is a number from 1 to 3; O-allyl, halogen, azido or amino;
A is, independently of each other, oxy, thioxy or methylene;
W is, independently of each other, oxo, thioxo or selenoxo;
V is, independently of each other, oxy, sulfanediyl or imino;
Y is, independently of each other, oxy, sulfanediyl, imino or methylene;
B is a base which is customary in nucleotide chemistry, with at least one B being a base which possesses an exocyclic amino group;
n is an integer from 1 to 100;
U is, independently of each other, hydroxyl, mercapto, BH₃, SeH, C₁-C₁₈-alkoxy, preferably C₁-C₆-alkoxy, C₁-C₁₈-alkyl, preferably C₁-C₆-alkyl, C₆-C₂₀-aryl, (C₆-C₁₄)-aryl- (C₁-C₈) -alkyl, NHR³, NR³R⁴ or a radical of the formula II
(OCH₂CH₂)ₚO(CH₂)_{q}CH₂R² (II)
in which
R³ is C₁-C₁₈-alkyl, preferably C₁-C₈-alkyl, C₆-C₂₀-aryl, (C₆-C₁₄)-aryl-(C₁-C₈)-alkyl, or -(CH₂)_{c}-[NH(CH₂)_{c}]_{d}-NR⁵R⁵, in which c is an integer from 2 to 6 and d is an integer from 0 to 6;
R⁵ is, independently of each other, hydrogen, C₁-C₆-alkyl or C₁-C₄-alkoxy-C₁-C₆-alkyl, preferably methoxyethyl;
R⁴ is C₁-C₁₈-alkyl, preferably C₁-C₈-alkyl and particularly preferably C₁-C₄-alkyl, C₆-C₂₀-aryl or (C₆-C₁₀)-aryl-(C₁-C₈)-alkyl, or, in the case of NR³R⁴, is, together with R³ and the nitrogen atom carrying them, a 5-6-membered heterocyclic ring which can additionally contain a further heteroatom from the group O, S and N;
P is an integer from 1 to 100, preferably from 3 to 10;
Q is an integer from 0 to 22, preferably from 0 to 15;
R² is hydrogen or a functional group such as hydroxyl, amino, NHR⁶, COOH, CONH₂, COOR⁷ or halogen, in which
R⁶ is C₁-C₄-alkyl, and
R⁷ is C₁-C₄-alkyl, preferably methyl;
Q and Q' are, independently of each other, hydrogen or conjugates which have a favorable effect on the properties of antisense oligonucleotides or of triple helix-forming oligonucleotides, or are used as the label for a DNA probe, or, in association with the hybridization of the oligonucleotide analog to the target nucleic acid, attack the latter while binding or cross-linking;
where
R¹ and the adjacent phosphoryl radical can be located either in the 2' and 3' positions or, conversely, in the 3' and 2' positions;
each nucleotide can be present in its D or L configuration;
the base B can be located in the alpha or beta position, with it also being possible for the oligonucleotide to contain 3'-3'- or 5'-5' inversions,
wherein
(a) a compound of the formula III 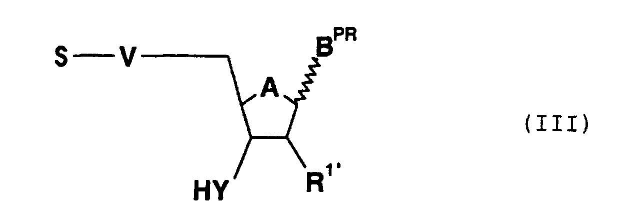 in which A, Y and V are defined as above, and
R^{1'} is defined as R¹ where, when R¹ is hydroxyl or amino, R^{1'} is a correspondingly protected group,
S is a S' protecting group which can be eliminated under acid conditions;
B^{PR} is a natural or unnatural nucleobase in which any exocyclic amino groups which may be present are protected by a cyclic diacyl group;
is reacted, in accordance with known methods, with a compound of the formula IV 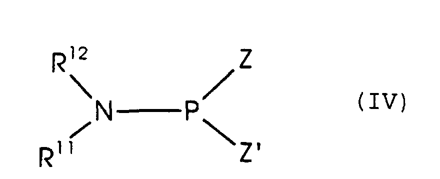 in which
Z' is OR¹³ or C₁-C₁₈-alkyl, C₁-C₁₈-alkoxy, C₆-C₂₀-aryl, C₆-C₁₄-aryl-C₁-C₈-alkyl, preferably OR¹³, C₁-C₆-alkyl, C₁-C₆-alkoxy, C₆-C₂₀-aryl, or C₆-C₁₄-aryl-C₁-C₈-alkyl, particularly preferably OR¹³;
R¹¹ and R¹² are identical or different and are C₁-C₈-alkyl, preferably isopropyl, or C₅-C₁₂-cycloalkyl, preferably up to C₈, benzyl or phenyl or, together with the nitrogen atom to which they are bonded, a saturated or unsaturated heterocyclic ring having, where appropriate, additional heteroatoms, for example morpholine, and substituents, such as OC(O)O-C₁-C₄-alkyl esters; and
R¹³ is a protecting group which can be eliminated with strong, nonnucleophilic bases,
Z is chlorine or bromine or a radical of the formula NR¹¹R¹², where R¹¹ and R¹² are defined as above;
in the presence of a base, preferably pyridine or a mixture of tetrahydrofuran (THF), dioxane, dichloromethane (DCM), chloroform, and/or acetonitrile with a C₁-C₄-trialkylamine, preferably trimethylamine, triethylamine or diisopropylethylamine, or, when Z is a radical of the formula NR¹¹R¹², then in the presence of a compound of the formula [HNR¹⁴R¹⁵R¹⁶]⁽⁺⁾A⁽⁻⁾,
where R¹⁴, R¹⁵ and R¹⁶ are identical or different and are a C₁-C₄-alkyl group and A is fluorine, chlorine or bromine, in particular chlorine;
or tetrazole or 5-(C₁-C₄-alkylthio)-1H-tetrazole, preferably in the presence of tetrazole or pyridine hydrochloride, to form a compound of the formula V 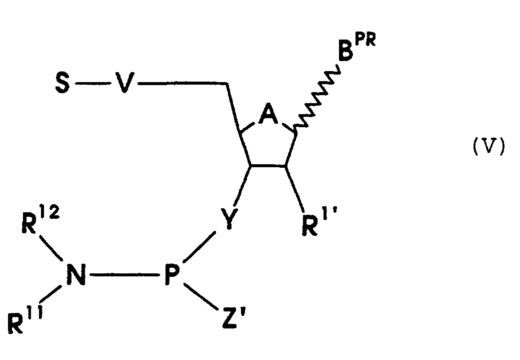 in which S, V, Y, A, B^{PR}, Z', R^{1'}, R¹¹ and R¹² are as defined above;
(b) compounds of the formula III are reacted, in accordance with known methods, with from 1 to 10 equivalents, preferably with from 1 to 2 equivalents, of a linker in a suitable organic solvent, where appropriate after adding a catalyst, to give a compound of the formula VI 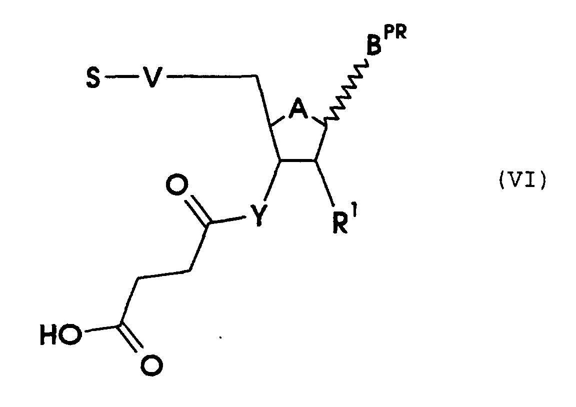 where S, V, Y, A, B^{PR} and R¹ are defined as above, and subsequently worked up in accordance with known methods, with the succinic acid residue in the 3' position serving as the linker to the polymer support which is employed in the synthesis.
(c) the compound of the formula VI is coupled, in accordance with known methods, to a solid support SS in a suitable solvent, with compounds of formula VII being obtained; 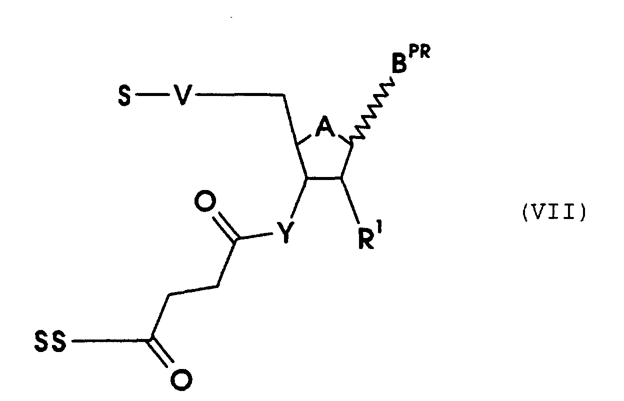
(d) the 5' protecting group is eliminated from VII in accordance with known methods;
(e) the resulting compound is reacted with a compound of the formula V in a suitable organic solvent, preferably acetonitrile, in the presence of a compound of the formula [HNR¹⁴R¹⁵R¹⁶]⁽⁺⁾ A⁽⁻⁾, which is defined as above, or tetrazole, 5-(C₁-C₄-alkylthio)-1H-tetrazole or 5-(C₆-C₁₂-aryl)-1H-tetrazole, preferably in the presence of tetrazole or pyridine hydrochloride, and
(f) the resulting compound is oxidized in accordance with known methods, where appropriate also in the presence of additional organic solvents,
(g) the reaction steps d - f are repeated until the desired chain length has been obtained;
(h) the compound which has been obtained in this way is deprotected by treating with DBU in a suitable organic solvent; and
(i) the oligonucleotide is cleaved from the support in accordance with known methods.

6. The process as claimed in claim 5, wherein unreacted compounds from step (d) are deactivated by means of a capping step.

7. The process as claimed in one of claims 5 or 6, wherein, in compounds of the formulae (III), (V), (VI) and (VII), B^{PR} is, independently of each other, 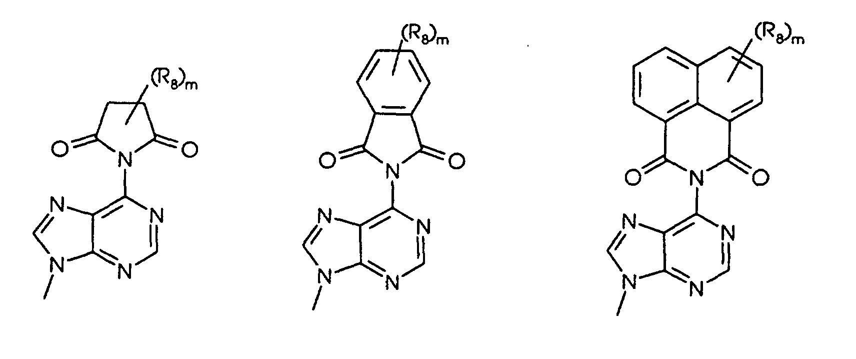 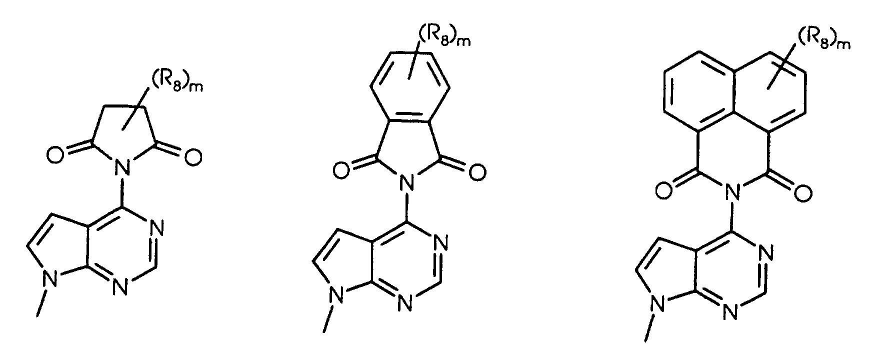 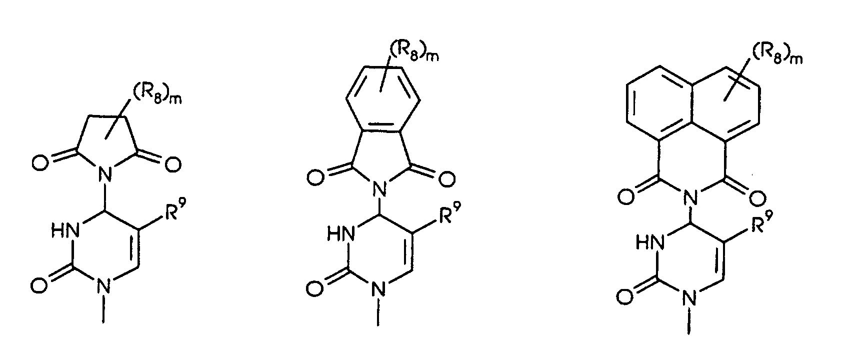 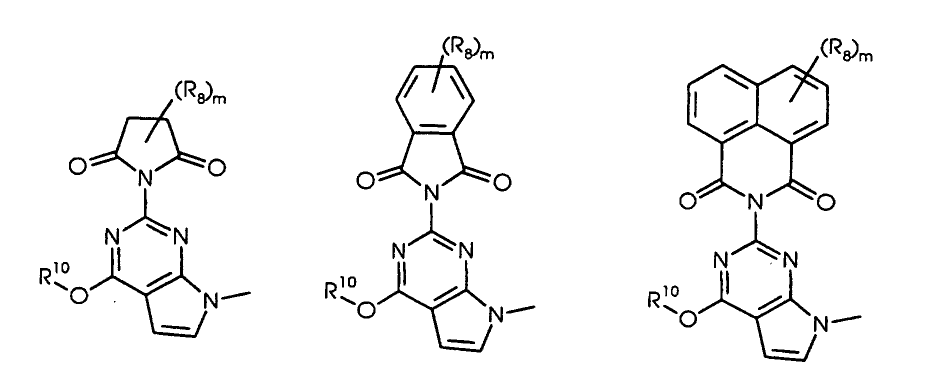 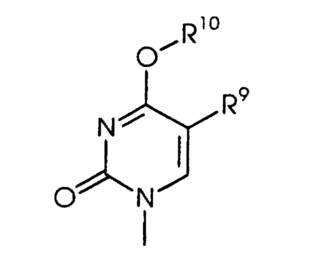 in which
m is a number from zero to four, preferably zero, and
R⁸ is, independently of each other, hydrogen, fluorine, chlorine, bromine, nitro, C₁-C₄-alkyl, C₁-C₄-alkoxy, CN,
R⁹ is, independently of each other, hydrogen, C₁-C₆-alkyl, C₂-C₆-(1-alkyne), preferably 1-propynyl and 1-hexynyl or fluorine, and
R¹⁰ is, independently of each other, hydrogen or a β-eliminatable protecting group.

8. The process as claimed in one of claims 5 to 7 for preparing a compound of the formula I in which
R¹ is hydrogen, hydroxyl, C₁-C₄-alkoxy or fluorine;
A is oxy;
W is oxo or thioxo;
V is oxy;
Y is oxy;
B is, independently of each other, adenine, cytosine, guanine, uracil, thymine, 5-propyneuracil, 5-propynecytosine, 5-hexyneuracil or 5-hexynecytosine
n is an integer from 5 to 40;
U is hydroxyl, mercapto, C₁-C₆-alkoxy, C₁-C₆-alkyl, NR³R⁴ or NHR³, in which
R³ is C₁-C₈-alkyl or methoxyethyl
R⁴ is C₁-C₈-alkyl, C₆-C₂₀-aryl or (C₆-C₁₀)-aryl-(C₁-C₈)-alkyl, or, in the case of NR³R⁴, is, together with R³ and the nitrogen atom carrying them, a 5-6-membered heterocyclic ring which can additionally contain a further heteroatom from the group O, S and N,
Q and Q' are, independently of each other, hydrogen.

9. The process as claimed in claim 8, wherein
R¹ is hydrogen
n is an integer from 5 to 30
U is hydroxyl or C₁-C₆-alkyl
Q and Q' are hydrogen
and the remaining variables are defined as in claim 7.

10. A compound of the formula V 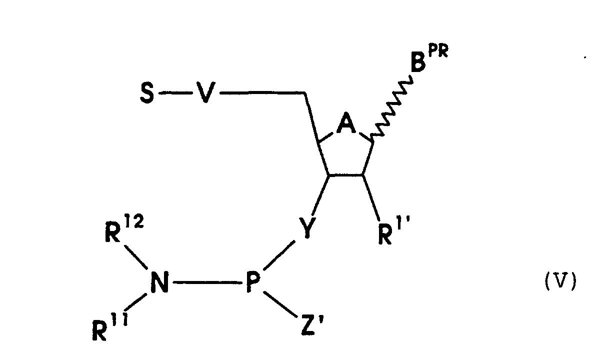 in which
R^{1'} is, independently of each other, hydrogen, C₁-C₁₈-alkoxy, where appropriate substituted one to three times by hydroxyl, or C₁-C₄-alkoxy, C₁-C₄-alkyl-O-(CH₂CH₂O)₁₋₃, O-allyl, fluorine, chlorine, azido or a protected hydroxyl group or amino group;
A is oxy, thioxy or methylene;
V is oxy, sulfanediyl or imino;
Y is oxy, sulfanediyl, imino or methylene;
S is a 5' protecting group which can be eliminated under acid conditions;
B^{PR} is a natural or unnatural nucleobase having an exocyclic amino group, with the exocyclic amino groups being protected by a cyclic diacyl group;
Z' is OR¹³, C₁-C₁₈-alkyl, C₁-C₁₈-alkoxy, C₆-C₂₀-aryl or C₆-C₁₄-aryl-C₁-C₈-alkyl;
R¹¹ and R¹² are identical or different and are C₁-C₈-alkyl, preferably isopropyl, or C₅-C₁₂-cycloalkyl, preferably up to C₈, benzyl or phenyl, or, together with the nitrogen atom to which they are bonded, a saturated or unsaturated heterocyclic ring having, where appropriate, additional heteroatoms, for example morpholine, and substituents, such as OC(O)O-C₁-C₄-alkyl esters; and
R¹³ is para-nitrophenylethyl or 2-cyanoethyl.

11. A compound of the formula V as claimed in claim 10, in which B^{PR} is 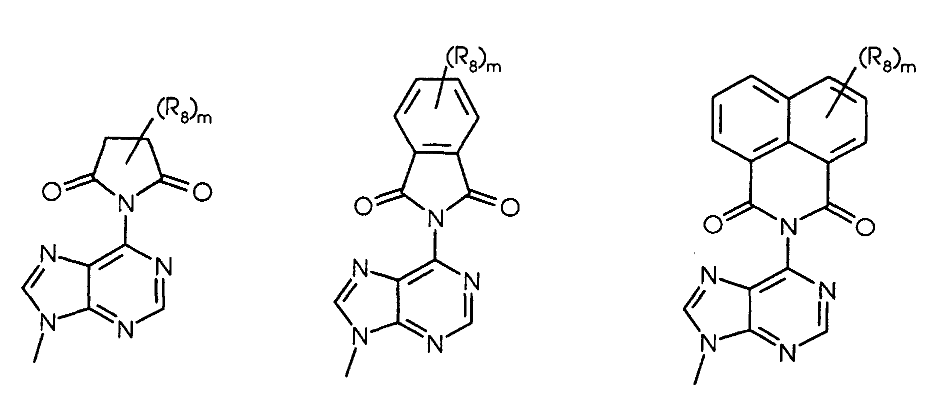 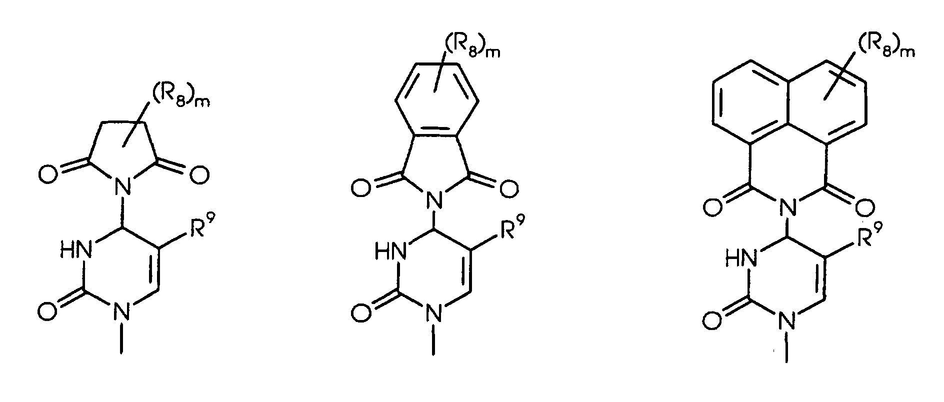 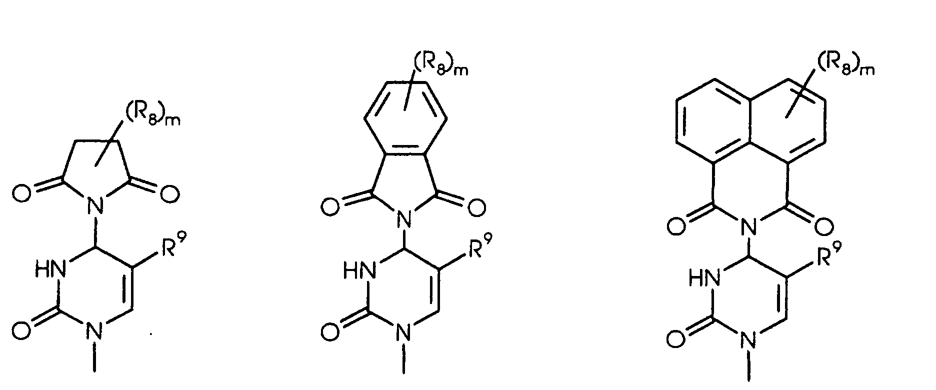 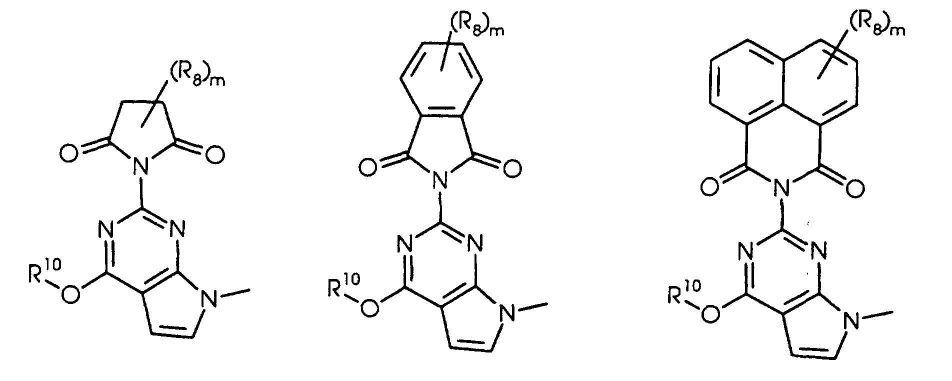 in which
m is a number from zero to four, preferably zero; and
R⁸ is, independently of each other, hydrogen, fluorine, chlorine, bromine, nitro, C₁-C₄-alkyl, C₁-C₄-alkoxy, or CN,
and R⁹ and R¹⁰ are as defined in claim 7.

12. A compound of the formula V as claimed in claim 10 or 11, in which
R^{1'} is hydrogen, a protected hydroxyl group, C₁-C₄-alkoxy or fluorine,
A is oxy;
V is oxy;
Y is oxy;
Z' is OR¹³, C₁-C₆-alkyl, C₁-C₆-alkoxy, C₆-C₂₀-aryl or C₆-C₁₄-aryl-C₁-C₈-alkyl, preferably OR¹³.
